# EUROPÄISCHE PATENTSCHRIFT

(11) **EP 4 021 541 B1**
(45) Veröffentlichungstag und Bekanntmachung des Hinweises auf die Patenterteilung: **09.07.2025**
(21) Anmeldenummer: 20800080.2
(22) Anmeldetag: 28.10.2020
(51) Int. Cl.: A61M 16/00, A61B 5/091

(54) **VENTILATION DEVICE**
BEATMUNGSVORRICHTUNG
DISPOSITIF DE VENTILATION

(30) Priorität: 31.10.2019 DE 102019129549
(43) Veröffentlichungstag der Anmeldung: 06.07.2022
(73) Patentinhaber: Hamilton Medical AG, 7402 Bonaduz (CH)
(72) Erfinder: REIDT, Sascha, 7206 Igis (CH); SCHRANZ, Christoph, 7306 Fläsch (CH); NOVOTNI, Dominik, 7000 Chur (CH)
(74) Vertreter: Ruttensperger Lachnit Trossin Gomoll
(86) Internationale Anmeldenummer: PCT/EP2020/080328
(87) Internationale Veröffentlichungsnummer: WO 2021/083981

(56) Entgegenhaltungen:
- DE-A1- 102017 101 645
- US-A- 5 915 381
- US-A1- 2013 074 844
- US-A1- 2019 275 276

## Beschreibung

Die vorliegende Erfindung betrifft eine Beatmungsvorrichtung zur künstlichen Beatmung eines Patienten, umfassend:
- eine Atemgas-Quellenanordnung, welche ein inspiratorisches Atemgas zur künstlichen Beatmung des Patienten bereitstellt,
- eine Strömungsveränderungsvorrichtung, welche dazu ausgebildet ist, einen inspiratorischen Atemgasfluss zu erzeugen und betragsmäßig zu verändern,
- eine Atemgasleitungsanordnung mit einem im Betrieb dem Patienten näher gelegenen proximalen Längsende und mit einem im Betrieb vom Patienten weiter entfernt gelegenen distalen Längsende, um den inspiratorischen Atemgasfluss von der Atemgas-Quellenanordnung zum Patienten hin zu fördern,
- eine Flusssensoranordnung, welche dazu ausgebildet ist, den inspiratorischen Atemgasfluss ebenso wie einen exspiratorischen Atemgasfluss betragsmäßig zu erfassen,
- eine Drucksensoranordnung, welche dazu ausgebildet ist, einen Druck des inspiratorischen Atemgases ebenso wie des exspiratorischen Atemgases in der Atemgasleitungsanordnung zu erfassen,
- eine Steuervorrichtung mit einem Datenspeicher, wobei die Steuervorrichtung signalübertragungsmäßig mit dem Datenspeicher, mit der Flusssensoranordnung und mit der Drucksensoranordnung verbunden ist und welche dazu ausgebildet ist, die Betriebsleistung der Strömungsveränderungsvorrichtung zur Veränderung des inspiratorischen Atemgasflusses zu steuern,
wobei die Steuervorrichtung dazu ausgebildet ist, die Strömungsveränderungsvorrichtung zur Durchführung eines P/V-Manövers anzusteuern, bei welchem einem Patienten in einer Inspirationsphase Atemgas unter Erhöhung des Atemgasdrucks zugeführt wird, welches in einer Exspirationsphase nach Abschluss der Druckerhöhung passiv aus dem Patienten ausströmt, wobei sowohl während der Inspirationsphase als auch während der Exspirationsphase für eine Vielzahl von Atemgas drücken das jeweils aufgrund des P/V-Manövers im Patienten vorhandene Manöver-Atemgasvolumen in Zuordnung zum herrschenden Atemgasdruck ermittelt wird.

Die vorliegende Erfindung betrifft außerdem ein Verfahren zur Durchführung eines P/V-Manövers an einer Patientenlunge, vorzugsweise zum Zwecke einer Ermittlung von Daten für eine Beurteilung einer Rekrutierbarkeit und Dehnbarkeit von Lungengewebe einer Patientenlunge.

Eine Beatmungsvorrichtung der eingangs genannten Art sowie ein Verfahren zur Durchführung eines P/V-Manövers an einer Patientenlunge sind aus der EP 2 091 429 B1 bekannt. Diese Druckschrift lehrt, einen positiven end-exspiratorischen Druck, im Folgenden in Übereinstimmung mit der Fachwelt als "PEEP" (für Positive End-Expiratory Pressure) bezeichnet, für einen konkreten Patienten durch ein sogenanntes P/V-Manöver an dem Patienten bzw. seiner Patientenlunge zu ermitteln.

Bei diesem aus der EP 2 091 429 B1 bekannten P/V-Manöver wird ausgehend von einem Start-Atemgasdruck unter fortwährender Erhöhung des Atemgasdrucks dem Patienten inspiratorisches Atemgas zugeführt bis ein vorbestimmter inspiratorischer Atemgasenddruck erreicht ist. Dabei wird eine inspiratorische P-V-Kurve aufgezeichnet, welche das während der Inspirationsphase des P/V-Manövers dem Patienten jeweils zugeführte inspiratorische Atemgasvolumen in Zuordnung zu dem jeweils herrschenden Atemgasdruck angibt.

Nachdem der vorbestimmte Atemgasenddruck erreicht ist, lässt man im Rahmen des immer noch fortdauernden P/V-Manövers das zuvor zugeführte Atemgas als exspiratorisches Atemgas aus der Patientenlunge entweichen, wiederum unter Erfassung des jeweils noch im Patienten vorhandenen Atemgasvolumens in Zuordnung zu dem bei der jeweiligen Erfassung herrschenden Druck des exspiratorischen Atemgases. Auch hierfür wird eine P-V-Kurve, diesmal als exspiratorische P-V-Kurve aufgezeichnet, welche das während der Exspirationsphase des P/V-Manövers im Patienten vorhandene Atemgasvolumen in Zuordnung zu dem während der Erfassung herrschenden exspiratorischen Atemgasdruck angibt. Das P/V-Manöver endet bei einem vorbestimmten Enddruck.

Die inspiratorische und die exspiratorische P-V-Kurve durchlaufen einen gemeinsamen Atemgasdruckbereich, wobei die beiden Kurven eine Hysterese aufweisen. Die exspiratorische P-V-Kurve weist über einen weiten mittleren Atemgasdruckbereich bei gleichen Atemgasdruckwerten höhere Volumenwerte auf.

Die EP 2 091 429 B1 lehrt, einen für den jeweiligen Patienten, an welchem das P/V-Manöver durchgeführt wird, passenden PEEP automatisiert als jenen Druck zu bestimmen, bei welchem die exspiratorische und die inspiratorische P-V-Kurve den betragsmäßig größten Unterschied aufweisen.

Da aufgrund der Charakteristik der beiden P-V-Kurven dann, wenn ein betragsmäßig größter Volumenunterschied zwischen den beiden P-V-Kurven erfasst wurde, bei weiter abnehmenden Druck des exspiratorischen Atemgases während der Exspirationsphase der Volumenunterschied zwischen der exspiratorischen und der inspiratorischen P-V-Kurve abnimmt und kein erneutes Ansteigen dieses Volumenunterschied zu erwarten ist, empfiehlt die EP 2 091 429 B1, die Exspirationsphase des P/V-Manövers abzubrechen, wenn während der Exspirationsphase ein betragsmäßig größter Volumenunterschied zwischen exspiratorischer und inspiratorischer P-V-Kurve erkannt werden konnte.

Aus der US 5,915,381 ist eine Beatmungsvorrichtung bekannt, welche lehrt, aus einer inspiratorischen P-V-Kurve, welche während einer normalen apparativen Beatmung eines Patienten ermittelt wird, eine momentane Compliance der beatmeten Patientenlunge zu berechnen und Beatmungsparameter, wie Druckniveaus, PEEP, Inspirationsdauer, Exspirationsdauer und Atmungsrate dann zu verändern, wenn die momentane Compliance der Lunge kleiner ist als ein vorbestimmter Schwellenwert.

Mit "Compliance" ist dabei in fachüblicher Weise ein elastischer Widerstand der Lunge gegen eine Volumenänderung bezeichnet. Sie ist in fachüblich bekannter Weise bestimmt durch das Verhältnis einer durch eine Druckänderung von Atemgas bewirkte Volumenänderung des Lungenvolumens. Mit "momentaner Compliance" ist im Gegensatz zu einer über eine vollständigen Inspirationsphase hinweg ermittelte Gesamt-Compliance oder mittlere Compliance eine zu einem konkreten Zeitpunkt bzw. bei einem konkreten Inspirationszustand herrschende Compliance bezeichnet.

Die US 5,915,381 verweist darauf, dass abhängig vom pathologischen Zustand der jeweils beatmeten Lunge unterschiedlich große Atemgasmengen der Lunge zugeführt werden müssen, um einen vollständigen oder teilweisen Kollaps der Lunge zu vermeiden. Eine kranke Lunge hat dabei in der Regel einen kleineren Beatmungsbereich als eine gesunde Lunge. Es muss daher gemäß US 5,915,381 für jeden Patienten ein spezifischer Compliance-Schwellenwert vorbestimmt werden, mit dem die momentane Compliance während einer apparativen bewirkten Inspirationsphase verglichen wird. Die US 5,915,381 nennt als eine zusätzlich mögliche Sicherheitsmaßnahme für einige Patienten, eine Inspirationsphase abzubrechen, sobald die während der Inspirationsphase ermittelte momentane Compliance unter einen vorbestimmten Schwellenwert fällt.

In der vorliegenden Anmeldung ist mit dem Begriff "Compliance", sofern im Einzelfall nichts Abweichendes angegeben ist, eine momentane Compliance bezeichnet.

Zu Beginn einer künstlichen Inspiration einer Lunge, in deren weitgehend ausgeatmetem Zustand, führt eine Erhöhung des inspiratorischen Atemgasdrucks zunächst zu einer verhältnismäßig geringen Volumenzunahme der Lunge. Die Compliance der Lunge ist daher zu Beginn der Inspirationsphase des P/V-Manövers betragsmäßig gering. Nach einer gewissen Füllung der Lunge mit inspiratorischem Atemgas nimmt das Volumen der Lunge mit Zunahme des Drucks des inspiratorischen Atemgases stärker zu als zu Beginn des P/V-Manövers, sodass in dieser zeitlich mittleren Phase des P/V-Manövers die Compliance der Lunge betragsmäßig größer ist als zu Beginn des Manövers. Gegen Ende des P/V-Manövers kann die bereits stark mit Atemgas gefüllte Lunge selbst unter weiterer Erhöhung des Drucks des inspiratorischen Atemgases nicht mehr volumenmäßig vergrößert werden, sodass gegen Ende eines P/V-Manövers die Compliance der Lunge betragsmäßig wieder abnimmt.

Die US 5,915,381 nennt daher den Vorteil, für jeden Patienten einen geeigneten Compliance-Schwellenwert zur Beeinflussung von Druckniveau, PEEP, Inspirationsdauer, Exspirationsdauer, Atemrate und dergleichen zu ermitteln, stellt diese Ermittlung eines vorbestimmten Compliance-Schwellenwerts jedoch der künstlichen Beatmung, während welcher der Compliance-Schwellenwert zur Anwendung kommt, voraus. Eine solche patientenspezifische Vorausberechnung eines Compliance-Schwellenwerts nach Anamnese des Patienten ist aufwendig und zeitintensiv. Weiterhin ist die Ermittlung eines patientenspezifischen Compliance-Schwellenwerts und Übermittlung desselben an die Beatmungsvorrichtung fehleranfällig.

Aus der DE 10 2017 101 645 A1 sind mehrere Beatmungsvorrichtungen und Beatmungsverfahren bekannt, welche eine Beatmung eines Patienten mit minimalem Energieeintrag in dessen Atemweg ermöglichen sollen. Gemäß einer aus der DE 10 2017 101 645 A1 bekannten Beatmungsvorrichtung soll hierzu sowohl der Inspirationsvorgang als auch der Exspirationsvorgang durch die Beatmungsvorrichtung derart gesteuert werden, dass sich zumindest über 60 % des Beatmungsintervalls das Verhältnis der bei druckgesteuerter Beatmung bei demselben Druckwert einmal während der Inspiration und einmal während der Exspiration auftretenden Compliances oder der bei volumengesteuerter Beatmung bei demselben Volumenwert wert einmal während der Inspiration und einmal während der Exspiration auftretenden Compliances einen Wert von zwischen 0,5 und 2 aufweisen.

Aus der US 2019/0275276 A1 ist eine Vorrichtung zum Aufzeichnen und Darstellen einer Druck-Volumen-Kurve während einer künstlichen Beatmung bekannt. Die Vorrichtung ist dazu ausgebildet, eine Steigung des Druckverlaufs zu ermitteln und darzustellen.

Aufgabe der vorliegenden Erfindung ist es, die aus der EP 2 091 429 B1 bekannte Beatmungsvorrichtung derart weiterzubilden, dass Patienten bei der Ausführung eines P/V-Manövers mit möglichst geringem Aufwand gut vor gesundheitlich nachteiligen Wirkungen des P/V-Manövers geschützt sind und dass aus dem P/V-Manöver erhaltene Messdaten in möglichst geringem Umfang durch Effekte einer Lungenüberdehnung belastet sind.

Diese Aufgabe löst die Erfindung durch eine Beatmungsvorrichtung der eingangs genannten Art, deren Steuervorrichtung dazu ausgebildet ist,
- während der Inspirationsphase des P/V-Manövers auf Grundlage von Signalen der Flusssensoranordnung und der Drucksensoranordnung eine Folge von Compliancewerten zu ermitteln, welche jeweils eine Lungen-Compliance der Lunge des Patienten repräsentieren,
- nach Maßgabe der Folge von Compliancewerten einen Bezugs-Compliancewert zu ermitteln,
- als Abbruchkriterium für die Inspirationsphase ausgehend von dem Bezugs-Compliancewert einen vom Bezugs-Compliancewert betragsmäßig verschiedenen Abbruch-Compliancewert als Schwellenwert zu bestimmen, und
- die Inspirationsphase dann abzubrechen, wenn der Abbruch-Compliancewert erreicht oder durchschritten wird.

Mithilfe der Flusssensoranordnung und der Drucksensoranordnung kann die Steuervorrichtung ohne Weiteres eine Folge von Compliancewerten ermitteln, welche jeweils eine Lungen-Compliance der Lunge des Patienten zu unterschiedlichen Zeitpunkten während der Durchführung des P/V-Manövers repräsentieren. Die Flusssensoranordnung misst einen Fluss, also Volumenstrom, an inspiratorischem Atemgas während der Inspirationsphase und an exspiratorischem Atemgas während der Exspirationsphase. Der Fluss oder Volumenstrom an Atemgas entspricht einer Änderung des Volumens an Atemgas in der Patientenlunge pro Zeiteinheit.

Das P/V-Manöver kann beispielsweise mit vorgegebener Drucksteigerungsrate des Atemgasdrucks oder mit vorgegebenem inspiratorischem Atemgas-Volumenstrom, etwa mit konstantem Atemgas-Volumenstrom erfolgen.

Die Flusssensoranordnung kann mehrere Flusssensoren aufweisen, etwa je einen für den inspiratorischen und für den exspiratorischen Atemgasfluss. Bevorzugt umfasst die Flusssensoranordnung nur einen Flusssensor, um sowohl den inspiratorischen als auch den exspiratorischen Atemgasfluss zu erfassen. Dieser ist bevorzugt proximal zwischen Atemgasleitungsanordnung und Patientenschnittstelle angeordnet, kann aber auch distal in einem Gehäuse der Beatmungsvorrichtung aufgenommen sein, in welchem beispielsweise auch die Strömungsveränderungsvorrichtung aufgenommen ist. Zur Erzielung von höherer Prozesssicherheit kann die Beatmungsvorrichtung auch mehrere Flusssensoren aufweisen, die jeweils sowohl den inspiratorischen als auch den exspiratorischen Atemgasfluss erfassen, etwa einen distalen Flusssensor in einem Gehäuse der Beatmungsvorrichtung und einen proximalen Flusssensor nahe beim Patienten. Entsprechendes gilt für die Drucksensoranordnung, die ebenfalls einen oder mehrere Drucksensoren aufweisen kann, um sowohl den Druck des inspiratorischen wie auch des exspiratorischen Atemgases zu messen.

Durch Integration des Flusses an Atemgas über eine Zeitspanne kann das Volumen an Atemgas ermittelt werden, welches während der Zeitspanne in der Atemgasleitungsanordnung geströmt ist. Dies ist auch das Volumen an Atemgas, welches in der Zeitspanne entweder als inspiratorisches Atemgas der Lunge zugeführt wurde oder als exspiratorisches Atemgas aus der Lunge abgeströmt ist.

Zur Unterscheidung von beliebigen anderen Atemgasvolumina, die während einer künstlichen Beatmung oder während eines P/V-Manövers auftreten können, ist in der vorliegenden Anmeldung das während eines P/V-Manövers durch Zufuhr von inspiratorischem Atemgas zur Patientenlunge und durch Abströmen von exspiratorischem Atemgas aus der Patientenlunge in der Patientenlunge vorhandene Atemgasvolumen als "Manöver-Atemgasvolumen" bezeichnet. Im Zweifel ist das Manöver-Atemgasvolumen während der Inspirationsphase das Integral des inspiratorischen Atemgasflusses über die Zeit während der Inspirationsphase und ist das Manöver-Atemgasvolumen während der Exspirationsphase der Wert des Integrals des inspiratorischen Atemgasflusses am Ende der Inspirationsphase abzüglich des Integrals des exspiratorischen Atemgasflusses über die Zeit während der Exspirationsphase. Das Manöver-Atemgasvolumen ist folglich darstellbar als Funktion der Zeit vom Beginn der Inspirationsphase bis zum Ende der Exspirationsphase. Da auch der Atemgasdruck in der Atemgasleitungsanordnung als Funktion der Zeit darstellbar ist, ist das Manöver-Atemgasvolumen auch darstellbar als Funktion des Atemgasdrucks in der Atemgasleitungsanordnung während der Inspirationsphase und während der Exspirationsphase.

Als P-V-Kurve oder P-V-Zusammenhang wird in der vorliegenden Anmeldung der funktionale Zusammenhang zwischen dem jeweils vorliegenden Manöver-Atemgasvolumen und dem währenddessen herrschenden Atemgasdruck bezeichnet.

Bevorzugt umfasst die Beatmungsvorrichtung auch eine Zeitmessvorrichtung, um Zeitdauern von Vorgängen und Teilvorgängen während des P/V-Manövers und Zeitpunkte während des P/V-Manövers ermitteln zu können. Mithilfe der Zeitmessvorrichtung kann auch eine Änderung des Atemgasdrucks, sei es nun der Druck eines inspiratorischen oder eines exspiratorischen Atemgases, genau ermittelt werden. Zwingend notwendig ist eine Zeitmessvorrichtung jedoch nicht. Beispielsweise kann die Steuervorrichtung dazu ausgebildet sein, Sensorsignale in vorbestimmten bekannten Zeitintervallen abzufragen, sodass zwischen den einzelnen abgefragten Erfassungswerten der Sensoranordnungen stets eine bekannte Zeitdauer verstreicht. Auch so kann eine zeitliche Änderung von relevanten Parametern, wie etwa Atemgasdruck oder Atemgasvolumen, ausreichend genau ermittelt werden.

Die so ermittelten Compliancewerte sind unmittelbar eine patientenspezifische Eigenschaft der Lunge des konkret beatmeten Patienten. Daher kann ohne großen Aufwand aus der Folge von Compliancewerten ein Bezugs-Compliancewert herangezogen werden, welcher für den jeweils beatmeten Patienten ebenfalls spezifisch ist.

Wird dann ausgehend von dem so ermittelten Bezugs-Compliancewert ein Abbruch-Compliancewert ermittelt, bei dessen Erreichen oder Durchschreiten die Inspirationsphase abgebrochen wird, kann verhindert werden, dass während des P/V-Manövers der Patient "überinspiriert" wird, also seine Lunge durch eine übergroße Menge an Atemgas oder/und durch einen übergroßen Atemgasdruck überdehnt oder allgemein belastet oder sogar beschädigt wird. Außerdem bewirkt eine Überdehnung der Lunge aufgrund des durch den Brustkorb begrenzten Volumens der die Lunge aufnehmenden Brusthöhle häufig eine nachteilige Belastung des Herz-Kreislauf-Systems des beatmeten Patienten. Denn die überdehnte Lunge fordert Raum zu Lasten von komprimierbarem Gewebe, wie etwa Blutgefäßen und dergleichen.

Somit kann die Steuervorrichtung aus den während der Inspirationsphase des P/V-Manövers sensorisch erhaltenen Daten für jeden Patienten einen passenden, individuellen Abbruch-Compliancewert als Schwellenwert für einen Abbruch der Inspirationsphase automatisiert ermitteln und verwenden. Eine aufwändige Anamnese des Patienten zur Ermittlung eines vorbestimmten Abbruch-Compliancewerts und ein möglicher Fehler bei dessen Übertragung an die Beatmungsvorrichtung können somit vermieden werden.

Die Steuervorrichtung kann daher dazu ausgebildet sein, die Inspirationsphase eines jeden P/V-Manövers patientenschonend bereits anhand des während des jeweiligen P/V-Manövers selbst ermittelten Abbruchkriteriums abzubrechen. Folglich kann die Steuervorrichtung weiter dazu ausgebildet sein, während der ununterbrochenen künstlichen Beatmung ein und desselben Patienten abhängig vom jeweiligen Gesundheitszustand der Patientenlunge betragsmäßig unterschiedliche Abbruch-Compliancewerte als Schwellenwert und Abbruchkriterium zur Anwendung zu bringen. Die Steuervorrichtung kann insbesondere dazu ausgebildet sein, während einer ununterbrochenen künstlichen Beatmung ein und desselben Patienten während jedes Atemhubs oder während jedes zweiten Atemhubs oder während jedes n-ten Atemhubs, wobei n eine ganze Zahl ist, einen Abbruch-Compliancewert zu ermitteln und für den Atemhub, in den die Ermittlung fällt, vorzugsweise bis zur Ermittlung eines nachfolgenden Abbruch-Compliancewerts, anzuwenden.

Grundsätzlich bestehen unterschiedliche Möglichkeiten, wie die Steuervorrichtung online, also während eines laufenden P/V-Manövers, aus den genannten Sensorsignalen die Folge von Compliancewerten ermitteln kann. Beispielsweise kann die Steuervorrichtung dazu ausgebildet sein, die Folge von Compliancewerten zu berechnen auf Grundlage eines Quotienten aus einem einem Atemgasdruck zugeordneten Volumenänderungswert und einem demselben Atemgasdruck zugeordneten Druckänderungswert, wobei der Volumenänderungswert eine zeitliche Veränderung des Manöver-Atemgasvolumens repräsentiert und wobei der Druckänderungswert eine zeitliche Veränderung des Atemgasdrucks repräsentiert. Hierzu genügen also die Erfassung von zwei Volumenwerten zu unterschiedlichen Zeitpunkten, aus welchen der Volumenänderungswert ermittelt werden kann, sowie die Erfassung von zwei Atemgasdruckwerten zu unterschiedlichen Zeitpunkten, vorzugsweise zu denselben Zeitpunkten, zu welchen die beiden Volumenwerte erfasst werden, sodass hieraus ein Druckänderungswert ermittelt werden kann. Dies ist eine Methode, die auf Differenzenquotienten beruht.

Alternativ, oder zur Bereitstellung einer Redundanz zusätzlich, kann die Steuervorrichtung dazu ausgebildet sein, die Folge von Compliancewerten zu berechnen auf Grundlage eines Quotienten aus einem einem Atemgasdruck zugeordneten Flusswert und dem demselben Atemgasdruck zugeordneten Druckänderungswert, wobei der Flusswert den inspiratorischen Atemgasfluss repräsentiert. Wie oben bereits dargestellt wurde, ist der Flusswert ein Maß für die Änderung des der Lunge zugeführten oder aus der Lunge entwichenen Atemgasvolumens pro Zeiteinheit. Auch dies ist eine Ermittlungsmethode auf Grundlage von Differenzenquotienten.

Dabei ist darauf zu achten, dass der Volumenänderungswert und der Druckänderungswert für eine übereinstimmende Zeitspanne, vorzugsweise für einen übereinstimmenden Zeitpunkt gelten, sodass der Volumenänderungswert und der Druckänderungswert einander zeitlich zugeordnet sind. Ändert sich der Atemgasdruck mit der Zeit und ändert sich das in der Patientenlunge vorhandene Atemgasvolumen mit der Zeit, so ändert sich wegen der Änderung des Atemgasdrucks das in der Patientenlunge vorhandene Abgasvolumen mit dem Atemgasdruck. Für den Flusswert und den Druckänderungswert gilt mutatis mutandis das gleiche.

Dabei kann eine gewisse zeitliche Unschärfe in der Übereinstimmung der Erfassungszeitpunkte der jeweiligen benötigten Sensorwerte hingenommen werden. Je besser jedoch die Erfassungszeitpunkte der benötigten Sensorwerte übereinstimmen, desto genauer ist allerdings die daraus berechnete Compliance.

Auch zur Bestimmung des Bezugs-Compliancewerts bestehen unterschiedliche Möglichkeiten.

Gemäß einer ersten bevorzugten Ausführungsform kann die Steuervorrichtung dazu ausgebildet sein, aus einer Folge von ab dem Start der Inspirationsphase des P/V-Manövers betragsmäßig zunächst größer und anschließend kleiner werdenden Compliancewerten den betragsmäßig größten Compliancewert als Bezugs-Compliancewert auszuwählen. Der ausgewählte Bezugs-Compliancewert wird vorteilhaft in dem Datenspeicher der Steuervorrichtung zwischengespeichert.

Betrachtet man den während eines P/V-Manövers beobachtbaren eindeutigen Zusammenhang des in der Patientenlunge vorhandenen Atemgasvolumens und des jeweils in der Atemgasleitungsanordnung herrschenden Atemgasdrucks, so ist die Compliance die erste Ableitung des sich mit dem Atemgasdruck ändernden Atemgasvolumens nach dem Atemgasdruck. Diese kann unter Verwendung diskreter Messpunkte in an sich bekannter Weise durch Differenzenquotienten ermittelt werden oder durch Interpolation diskreter Messpunkte durch eine differenzierbare Funktion und durch Ableitung derselben, um nur zwei Verfahren zu nennen.

Einfacher ausgedrückt: die Compliance ist die Steigung des Graphen des inspiratorischen Manöver-Atemgasvolumens als Funktion des Atemgasdrucks. Aufgrund der eingangs dargestellten Änderung der Compliance während der Inspirationsphase eines P/V-Manövers weist der Graph des Manöver-Atemgasvolumens als Funktion des Atemgasdrucks einen näher beim Start-Atemgasdruck des P/V-Manövers gelegenen Abschnitt mit konkaver Krümmung und einen näher beim Enddruck des P/V-Manövers gelegenen Abschnitt mit konvexer Krümmung auf. Üblicherweise weist der Wendepunkt am Übergang zwischen diesen beiden Abschnitten mit unterschiedlichem Krümmungssinn eine betragsmäßig sehr große oder sogar die betragsmäßig größte Compliance der inspiratorischen P-V-Kurve auf. Start-Atemgasdruck und Enddruck des P-V-Manövers können die betragsmäßig gleichen Drücke sein. Sie können jedoch auch verschieden sein, wobei dann bevorzugt der Enddruck ein niedrigerer Absolutdruck des Atemgases ist als der Start-Atemgasdruck. Grundsätzlich kann der Start-Atemgasdruck im Rahmen des medizinisch Sinnvollen frei gewählt werde. Bevorzugt ist als Start-Atemgasdruck ein Druck ausgewählt, welcher in einem Druckbereich gelegen ist, der von dem für den jeweiligen Patienten ermittelten PEEP bis etwa zum 1,6-Fachen des PEEP reicht, Bereichsgrenzen eingeschlossen.

Daher kann zusätzlich oder alternativ die Steuervorrichtung dazu ausgebildet sein, aus einer Folge von Wertepaaren aus Atemgasdruck und dem jeweiligen Atemgasdruck zugeordnetem inspiratorischen Manöver-Atemgasvolumen einen Wendepunkt zwischen mit unterschiedlichem Krümmungssinn gekrümmten Abschnitten eines die Folge von Wertepaaren repräsentierenden Graphen zu ermitteln und den dem Atemgasdruck am Wendepunkt zugeordneten Compliancewert als Bezugs-Compliancewert auszuwählen. Zur Ermittlung des Wendepunktes kann die Steuervorrichtung dazu ausgebildet sein, in an sich bekannter Weise die zweite Ableitung der Volumenwerte als Funktion der Druckwerte nach dem Druck zu bilden. Dies kann gemäß einer Alternative, wie die Ermittlung der ersten Ableitung, durch Interpolation erhaltener Messwerte mit einer zweifach differenzierbaren Funktion und durch Ableitung derselben erfolgen. Die Bezugs-Compliance liegt dann bei demjenigen Atemgasdruckwert, bei welchem die zweite Ableitung Folge von Wertepaaren den Wert Null oder einen dem Wert Null betragsmäßig nächstgelegenen Wert aufweist. Die Bildung der zweiten Ableitung kann gemäß einer weiteren Alternative, ebenso wie die Ermittlung der ersten Ableitung, durch Differenzenquotienten erfolgen.

Bevorzugt bildet die Folge von den genannten Wertepaaren eine P-V-Kurve.

Durch die beiden oben genannten bevorzugten Arten zur Auswahl bzw. Bestimmung eines Bezugs-Compliancewerts wird zum einen ein Bezugs-Compliancewert ausgewählt, welcher erfahrungsgemäß in einem zeitlichen Mittelbereich der Inspirationsphase des P/V-Manövers gelegen ist, zu welchem mit an Sicherheit grenzender Wahrscheinlichkeit eine Überbelastung der Lunge durch die künstliche Inspiration nicht vorliegt. Außerdem sind ausgehend von dem so ausgewählten Bezugs-Compliancewert die während der weiteren Inspirationsphase auftretenden Compliancewerte betragsmäßig kleiner als der Bezugs-Compliancewert, sodass ausgehend von dem Bezugs-Compliancewert ein aussagekräftiger Abbruch-Compliancewert mit großer Sicherheit erhalten werden kann.

Zur Ermittlung des Abbruch-Compliancewerts kann die Steuervorrichtung dazu ausgebildet sein, den Abbruch-Compliancewert durch Multiplikation des Bezugs-Compliancewerts mit einem vorbestimmten Faktor oder durch Addition des Bezugs-Compliancewerts mit einem vorbestimmten Summanden zu berechnen. Der vorbestimmte Summand ist bevorzugt negativ, da der Abbruch-Compliancewert aufgrund der oben geschilderten Natur des Verlaufs des Compliancewerts während der Inspirationsphase betragsmäßig in der Regel kleiner ist als der als Bezugs-Compliancewert bevorzugte maximale Compliancewert. Aus demselben Grund ist der vorbestimmte Faktor bevorzugt kleiner als 1.

In einem bevorzugten Ausführungsbeispiel kann der Abbruch-Compliancewert 75 % bis 95 %, vorzugsweise 80 % bis 92,5%, besonders bevorzugt 85 % bis 91 % des Bezugs-Compliancewerts betragen. Besonders bevorzugt beträgt der Abbruch-Compliancewert etwa 90 % des Bezugs-Compliancewerts, wenn dieser in der Nähe des maximal auftretenden Compliancewerts gewählt ist, also etwa in einem Bereich von 40 % bis 60 % des maximal auftretenden Compliancewerts. Dies gilt unabhängig von der Art und Weise der Berechnung des Abbruch-Compliancewerts ausgehend vom Bezugs-Compliancewert. So kann eine Überbelastung, insbesondere Überdehnung, der Patientenlunge während der Inspirationsphase sicher vermieden werden.

Grundsätzlich kann daran gedacht sein, das P/V-Manöver, wie aus dem Stand der Technik bekannt, zur Ermittlung eines für den jeweiligen beatmeten Patienten besonders geeigneten PEEP durchzuführen.

Es hat sich jedoch gezeigt, dass durch das P/V-Manöver eine belastbare und zuverlässige Aussage über den zu erwartenden Erfolg eines Recruitment-Verfahren zur Rekrutierung von funktionsfähigem Lungenvolumen erhalten werden kann.

Dabei ist die Verwendung des oben genannten Abbruchkriteriums des Abbruch-Compliancewerts von besonderem Vorteil, weil dadurch die Heranziehung von Werten zur Beurteilung des zu erwartenden Erfolgs eines Recruitment-Verfahren vermieden werden kann, die während einer Überlastung der Lunge erfasst worden wären und die daher bei ihrer Einbeziehung in die Beurteilung das Beurteilungsergebnis nachteilig verfälschen würden.

Abweichend von und alternativ zu der zuvor beschriebenen Art und Weise der Vermeidung einer Erfassung von Werten während einer Lungenüberlastung, insbesondere Lungenüberdehnung, durch die automatisierte Bestimmung eines Abbruch-Compliancewerts, kann die unerwünschte Erfassung von Werten während einer Lungenüberdehnung gemäß weniger bevorzugter Ausführungsformen auch wie folgt erzielt werden:
Gemäß einer ersten Alternative kann ein vorbestimmter negativer Wert der zweiten Ableitung der P/V-Kurve als Abbruchkriterium dienen. Die zweite Ableitung kann durch die Steuervorrichtung wie oben beschrieben bestimmt werden. Als wenigstens ein weiteres Zusatzkriterium kann das Abbruchkriterium von der Steuervorrichtung erst nach einem Nulldurchgang der zweiten Ableitung oder nach zuvor erfassten positiven Werten der zweiten Ableitung aktiviert werden.

Gemäß einer zweiten Alternative kann zunächst ein erstes P/V-Manöver ohne Ermittlung eines inspiratorischen Abbruchkriteriums durchgeführt werden, wie man es aus dem Stand der Technik kennt. Wird während dieses ersten P/V-Manövers eine Lungenüberdehnung festgestellt, kann die Steuervorrichtung dazu ausgebildet sein, ein zweites P/V-Manöver mit reduziertem Enddruck durchzuführen. Wird während des ersten P/V-Manövers keine Lungenüberdehnung festgestellt, werden die während des ersten P/V-Manövers erhaltenen Werte oder/und die erhaltene P-V-Kurve zur Beurteilung der Rekrutierbarkeit der Lunge verwendet, ansonsten werden die während des zweiten P/V-Manövers erhaltenen Werte oder/und die erhaltene P-V-Kurve verwendet.

Gemäß einer dritten Alterative ist die Steuervorrichtung dazu ausgebildet, einen von einer Bedienperson oder aus einer abrufbaren Datenquelle für ein P/V-Manöver erhaltenen Enddruck um ein vorbestimmtes Maß zu verringern, entweder durch Subtraktion eines vorbestimmten Sicherheits-Margenwerts oder durch Multiplikation mit einem Wert kleiner als 1, vorzugsweise mit einem Wert zwischen 0,78 und 0,92, etwa mit 0,8.

Die Steuervorrichtung kann zur Ausführung einer der drei genannten Alternativen ausgebildet sein.

Die zuverlässige Beurteilung der Erfolgsaussichten eines Recruitment-Verfahren unmittelbar aus Daten eines P/V-Manövers ist deshalb so vorteilhaft, weil sie die Durchführung einer computertomografischen Untersuchung ersetzen kann. Der beatmete Patient kann hinsichtlich der Erfolgsaussichten eines Recruitment-Verfahrens im Falle einer wenigstens teilweise kollabierten Patientenlunge folglich unmittelbar in seinem Krankenbett untersucht werden. Das Recruitment-Verfahren kann jedes beliebige in der medizinischen Fachwelt bekannte Recruitment-Verfahren sein.

Zur Bereitstellung der für die Beurteilung der Erfolgsaussichten eines Recruitment-Verfahrens des jeweils beatmeten Patienten notwendigen Daten kann die Steuervorrichtung dazu ausgebildet sein, einen Volumen-Quotientenwert zu berechnen aus
- dem betragsmäßig größten während des P/V-Manövers für einen Atemgasdruck auftretenden Unterschied zwischen dem exspiratorischen und dem inspiratorischen Manöver-Atemgasvolumen und
- einem Unterschiedswert zwischen einem Manöver-Atemgasvolumenwert in einem oberen Endbereich und einem Manöver-Atemgasvolumenwert in einem unteren Endbereich des während des P/V-Manövers durchlaufenden Atemgasdruckbereichs,
wobei der untere Endbereich einen Start-Atemgasdruck enthält, bei welchem das P/V-Manöver beginnt, und bis zum 1,05-Fachen des Start-Atemgasdruck reicht, und wobei der obere Endbereich einen Abbruch-Atemgasdruck enthält, dem der Abbruch-Compliancewert zugeordnet ist, und bei 95 % des Abbruch-Atemgasdrucks beginnt.

Zur schließlichen Beurteilung der Erfolgsaussichten eines späteren Recruitment-Verfahrens kann, muss aber nicht, die Steuervorrichtung dazu ausgebildet sein, dann, wenn der Volumen-Quotientenwert einen vorbestimmten ersten Schwellenwert übersteigt, eine Ausgabe zu erzeugen, welche anzeigt, dass ein Recruitment-Verfahren zur Rekrutierung der Patientenlunge überwiegend Aussicht auf Erfolg hat. Dieser vorbestimmte erste Schwellenwert liegt vorzugsweise zwischen 38 % und 46 %, wobei sich in bisherigen Untersuchungen 42 % als geeignetster erster Schwellenwert erwiesen hat, um eine voraussichtlich erfolgreich rekrutierbare Patientenlunge von einer voraussichtlich nicht erfolgreich rekrutierbaren Patientenlunge zu unterscheiden.

Alternativ oder zusätzlich kann die Steuervorrichtung zur Bereitstellung der für die Beurteilung der Erfolgsaussichten eines Recruitment-Verfahrens des jeweils beatmeten Patienten notwendigen Daten dazu ausgebildet sein, einen Hysterese-Quotientenwert zu berechnen aus
- dem Flächeninhalt der Hysterese-Fläche, welche die Graphen der exspiratorischen und der inspiratorischen Manöver-Atemgasvolumina als Funktionen des Atemgasdrucks zwischen einem Start-Atemgasdruck, bei welchem das P/V-Manöver beginnt, und einen Abbruch-Atemgasdruck, dem der Abbruch-Compliancewert zugeordnet ist, und
- dem Flächeninhalt eines die Hysterese-Fläche umschreibenden Rechtecks, dessen eine Ecke bestimmt ist durch einen unteren Atemgasdruckwert in einem unteren Endbereich des während des P/V-Manövers durchlaufenden Atemgasdruckbereichs und durch den dem unteren Atemgasdruckwert zugeordneten Atemgasvolumenwert,
wobei der untere Endbereich den Start-Atemgasdruck enthält, und bis zum 1,05-Fachen des Start-Atemgasdruck reicht, und dessen diagonal gegenüberliegende Ecke bestimmt ist durch einen oberen Atemgasdruckwert in einem oberen Endbereich und durch den dem oberen Atemgasdruckwert zugeordneten Atemgasvolumenwert, wobei der obere Endbereich einen Abbruch-Atemgasdruck enthält, dem der Abbruch-Compliancewert zugeordnet ist, und bei 95 % des Abbruch-Atemgasdrucks beginnt.

Zur Beurteilung der Erfolgsaussichten eines Recruitment-Verfahrens aus den zuvor genannten Daten kann, muss aber nicht, die Steuervorrichtung dazu ausgebildet ist, dann, wenn der Hysterese-Quotientenwert einen vorbestimmten zweiten Schwellenwert übersteigt, eine Ausgabe zu erzeugen, welche anzeigt, dass ein Recruitment-Verfahren zur Rekrutierung der Patientenlunge überwiegend Aussicht auf Erfolg hat. Bevorzugt ist der zweite Schwellenwert vom ersten Schwellenwert betragsmäßig verschieden. Für den Hysterese-Quotientenwert hat sich ein zweiter Schwellenwert in einem Bereich von 28 % bis 36 % als aussagekräftig erwiesen. Bevorzugt liegt aufgrund bisheriger Untersuchungen der zweite Schwellenwert bei 32 %.

Es ist nachzutragen, dass die Atemgas-Quellenanordnung der Beatmungsvorrichtung als eine Atemgasquelle eine Ansaugöffnung aufweisen kann, durch welche hindurch Umgebungsluft oder Gas aus einem vorbestimmten Gasvorrat angesaugt werden kann. Die Atemgas-Quellenanordnung kann zusätzlich oder alternativ einen Gasvorrat als Atemgasquelle aufweisen, beispielsweise als Vorratsbehälter oder als Anschlussformation zum Anschluss einer Versorgungsleitung, welche die Beatmungsvorrichtung mit einem lokal installierten Gasvorrat verbindet, wie dies häufig in Kliniken der Fall ist. Um die Möglichkeit bereitzustellen, unterschiedliche Gase zu einem Atemgas zu mischen, kann die Atemgas-Quellenanordnung eine Mehrzahl von einzelnen Atemgasquellen, wie die oben genannten, aufweisen. Dabei können die unterschiedlichen zu mischenden Gase aufgrund von Bereitstellung und Entspannung unterschiedliche Temperaturen oder/und unterschiedliche Feuchtigkeit aufweisen. Um sicher zu gehen, dass das inspiratorische Atemgases den Patienten mit einer einmal eingestellten Feuchtigkeit tatsächlich erreicht, wird besonders bevorzugt in Inspirationsrichtung stromabwärts einer bevorzugt vorhandenen Befeuchtungsvorrichtung kein Atemgasbestandteil mehr dem aus der Befeuchtungsvorrichtung austretenden Atemgasfluss zugefügt.

Nachfolgend wird ein Verfahren zur Durchführung eines P/V-Manövers an einer Patientenlunge beschrieben, insbesondere zur Ermittlung von Daten für eine Beurteilung einer Rekrutierbarkeit von Lungengewebe, wobei das Verfahren die folgenden Schritte umfasst:
- Durchführen eines P/V-Manövers und dabei Zuführen von inspiratorischem Atemgas zu einem Patienten in einer Inspirationsphase unter Erhöhung des Atemgasdrucks,
- während der Inspirationsphase: Ermitteln eines während der Inspirationsphase zugeführten inspiratorischen Manöver-Atemgasvolumens oder eines inspiratorischen Manöver-Volumenstroms an inspiratorischem Atemgas und Ermitteln des Atemgasdrucks,
- Ermitteln einer Folge von Compliancewerten, welche jeweils eine Lungen-Compliance der Lunge des Patienten repräsentieren,
- Ermitteln eines Bezugs-Compliancewerts aus der Folge von Compliancewerten nach Maßgabe der Folge von Compliancewerten,
- Bestimmen, ausgehend von dem Bezugs-Compliancewert, eines vom Bezugs-Compliancewert betragsmäßig verschiedenen Abbruch-Compliancewerts als Abbruch-Schwellenwert, und
- Abbrechen der Inspirationsphase dann, wenn der Abbruch-Compliancewert erreicht oder durchschritten wird.

Bevorzugt betrifft die vorliegende Erfindung auch eine Vorrichtung, die zur Ausführung des zuvor genannten Verfahrens ausgebildet ist. Dies ist bevorzugt die zuvor beschriebene und weitergebildete Beatmungsvorrichtung.

Bei der Beschreibung der Beatmungsvorrichtung dargelegte Verfahrensaspekte sind Weiterbildungen des Verfahrens und bei der Beschreibung des Verfahrens dargelegte Vorrichtungsaspekte sind Weiterbildungen der erfindungsgemäßen Beatmungsvorrichtung.

Durch das Verfahren kann, wie bereits zuvor beschriebenen, eine Überlastung der Patientenlunge durch übermäßig hohen Atemgasdruck verhindert werden. Mit der Verhinderung der Überlastung, insbesondere Überdehnung der Lunge, wird auch die Erfassung von Messwerten oder Messwertepaaren in einem Überlastungszustand der Lunge vermieden. In einem Überlastungszustand erfasste Folgen von Messwertepaaren haben, verglichen mit in einem Normal-Belastungszustand der Lunge erfassten Wertepaaren nur eine eingeschränkte Aussagekraft oder verfälschen ein Messergebnis sogar, zu dem sie gehören.

Um aus dem P/V-Manöver Daten für die weitere Behandlung eines beatmeten Patienten gewinnen zu können, schließt sich bevorzugt zeitlich an die Inspirationsphase eine Exspirationsphase an, in welcher Atemgas passiv aus dem Patienten ausströmt, wobei während der Exspirationsphase sowohl der exspiratorische Atemgasdruck als auch ein exspiratorischer Manöver-Atemgasvolumenwert bestimmt werden, wobei der exspiratorische Manöver-Atemgasvolumenwert ein aufgrund des P/V-Manövers während der Exspirationsphase im Patienten vorhandenes exspiratorisches Manöver-Atemgasvolumen repräsentiert.

Sowohl bei dem beschriebenen Verfahren als auch bei der erfindungsgemäßen Vorrichtung, insbesondere Beatmungsvorrichtung, werden während des P/V-Manövers Manöver-Atemgasvolumenwerte in Zuordnung zu einem Atemgasdruck als Wertepaare bzw. als Wertzusammenhänge erfasst und gespeichert. Einem Manöver-Atemgasvolumenwert wird dabei der während seiner Erfassung herrschende Atemgasdruck zugeordnet.

Während die Bestimmung des inspiratorischen Manöver-Atemgasvolumenwerts vergleichsweise einfach ist, beispielsweise durch Integration der Flusswerte seit Beginn der Inspirationsphase, erfordert die Bestimmung des exspiratorischen Manöver-Atemgasvolumenwerts einen höheren Rechenaufwand. Das Bestimmen des exspiratorischen Manöver-Atemgasvolumenwerts im Rahmen des Verfahrens kann beispielsweise umfassen:
- ein Bestimmen eines während der Exspirationsphase ausgeatmeten Volumens an exspiratorischem Atemgas oder/und
- ein Bestimmen eines exspiratorischen Manöver-Volumenstroms an exspiratorischem Atemgas.

Der exspiratorische Manöver-Atemgasvolumenwert kann dann ermittelt werden aus dem inspiratorischen Manöver-Atemgasvolumenwert am Ende der Inspirationsphase abzüglich entweder des bestimmten ausgeatmeten Volumens oder des über die bisherige Dauer der Exspirationsphase integrierten Manöver-Volumenstroms.

Weiterhin kann das Verfahren zur Ermittlung von Daten für eine spätere Beurteilung der Erfolgsaussichten eines Recruitment-Verfahrens an der jeweiligen Patientenlunge umfassen: ein Berechnen eines Volumen-Quotientenwerts aus dem betragsmäßig größten während des P/V-Manövers für einen Atemgasdruck auftretenden Unterschied zwischen dem exspiratorischen und dem inspiratorischen Manöver-Atemgasvolumen und einem Bezugs-Manöver-Atemgasvolumen in einem oberen Endbereich des während des P/V-Manövers durchlaufenden Atemgasdruckbereichs, wobei der obere Endbereich einen Abbruch-Atemgasdruck enthält, dem der Abbruch-Compliancewert zugeordnet ist, und bei 95 % des Abbruch-Atemgasdrucks beginnt. Es gilt hier insbesondere das oben im Zusammenhang mit der erfindungsgemäßen Beatmungsvorrichtung zum Volumen-Quotientenwert Gesagte.

Alternativ oder zusätzlich kann das Verfahren zur Ermittlung von Daten für eine spätere Beurteilung der Erfolgsaussichten eines Recruitment-Verfahrens an der jeweiligen Patientenlunge umfassen: ein Berechnen eines Hysterese-Quotientenwerts aus
- dem Flächeninhalt der Hysterese-Fläche, welche die Graphen der exspiratorischen und der inspiratorischen Manöver-Atemgasvolumina als Funktionen des Atemgasdrucks zwischen dem Start-Atemgasdruck, bei welchem das P/V-Manöver beginnt, und einen Abbruch-Atemgasdruck, dem der Abbruch-Compliancewert zugeordnet ist, und
- dem Flächeninhalt eines die Hysterese-Fläche umschreibenden Rechtecks, dessen eine Ecke bestimmt ist durch einen unteren Atemgasdruckwert in einem unteren Endbereich des während des P/V-Manövers durchlaufenden Atemgasdruckbereichs und durch den dem unteren Atemgasdruckwert zugeordneten Atemgasvolumenwert,
wobei der untere Endbereich den Start-Atemgasdruck enthält, und bis zum 1,05-Fachen des Start-Atemgasdruck reicht, und dessen diagonal gegenüberliegende Ecke bestimmt ist durch einen oberen Atemgasdruckwert in einem oberen Endbereich und durch den dem oberen Atemgasdruckwert zugeordneten Atemgasvolumenwert, wobei der obere Endbereich einen Abbruch-Atemgasdruck enthält, dem der Abbruch-Compliancewert zugeordnet ist, und bei 95 % des Abbruch-Atemgasdrucks beginnt.

Weiter kann das Verfahren, wie oben im Zusammenhang mit der Beatmungsvorrichtung beschrieben, nach der Datenermittlung einen Schritt einer Datenauswertung zur Beurteilung der Erfolgsaussichten eines Recruitment-Verfahren aufweisen. Hierzu kann das Verfahren einen Schritt eines Vergleichs des Volumen-Quotientenwerts mit einen vorbestimmten ersten Schwellenwert aufweisen, wobei abhängig vom Vergleichsergebnis eine Ausgabe erzeugt wird, welche anzeigt, ob ein Recruitment-Verfahren zur Rekrutierung der Patientenlunge überwiegend Aussicht auf Erfolg hat oder nicht. Für den vorbestimmten ersten Schwellenwert gilt das oben Gesagte. Bevorzugt wird dann, wenn der Volumen-Quotientenwert einen den ersten Schwellenwert übersteigenden Betrag aufweist, eine Ausgabe erzeugt, welche anzeigt, dass das Recruitment-Verfahren überwiegend Aussicht auf Erfolg hat. Vorzugsweise wird andernfalls keine Anzeige oder eine Anzeige gegenteiligen Inhalts ausgegeben.

Zur Beurteilung der Erfolgsaussichten eines Recruitment-Verfahrens aus dem zuvor genannten Hysterese-Quotientenwert kann das Verfahren den Schritt eines Vergleichs des Hysterese-Quotientenwert mit einem vorbestimmten zweiten Schwellenwert aufweisen, wobei abhängig vom Vergleichsergebnis eine Ausgabe erzeugt wird, welche anzeigt, ob ein Recruitment-Verfahren zur Rekrutierung der Patientenlunge überwiegend Aussicht auf Erfolg hat oder nicht. Für zweiten Schwellenwert gilt wiederum das oben Gesagte. Bevorzugt liegt aufgrund bisheriger Untersuchungen der zweite Schwellenwert bei 32 %. Bevorzugt wird dann, wenn der Hysterese-Quotientenwert den vorbestimmten zweiten Schwellenwert übersteigt, eine Ausgabe erzeugt, welche anzeigt, dass ein Recruitment-Verfahren zu Rekrutierung der Patientenlunge überwiegend Aussicht auf Erfolg hat.

Die vorliegende Erfindung mit nachfolgend anhand der beiliegenden Zeichnungen näher dargestellt. Es stellt dar:
- Figur 1: eine schematische Darstellung einer erfindungsgemäßen, zur künstlichen Beatmung eines Patienten hergerichteten Beatmungsvorrichtung, und
- Figur 2: eine schematische Darstellung einer durch ein P/V-Manöver der erfindungsgemäßen Beatmungsvorrichtung ermittelten P-V-Kurve sowie ihrer Auswertung.

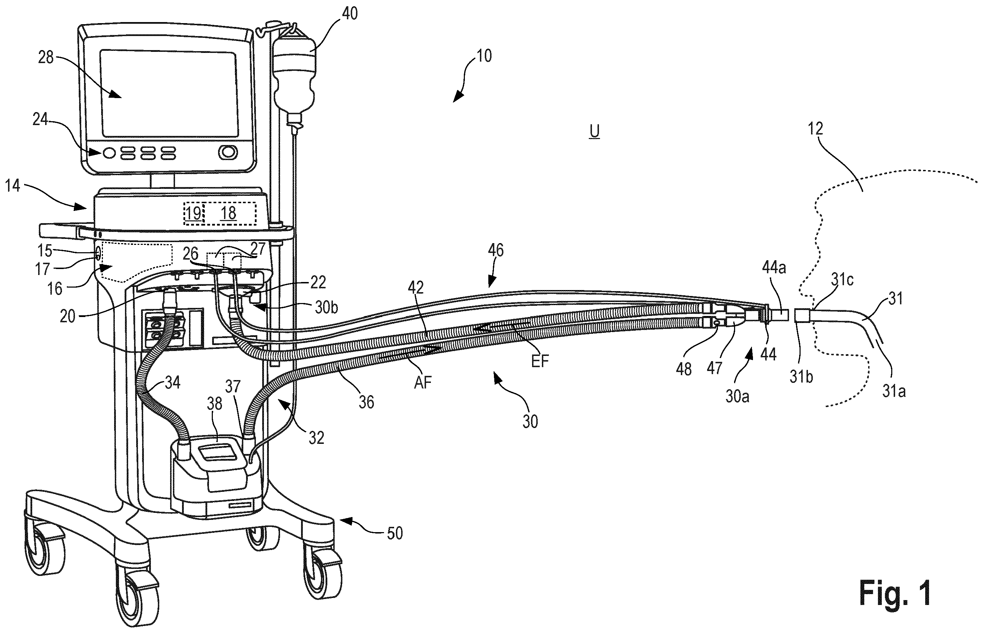

In Figur 1 ist eine erfindungsgemäße Ausführungsform einer Beatmungsvorrichtung allgemein mit 10 bezeichnet. Die Beatmungsvorrichtung 10 dient im dargestellten Beispiel zur künstlichen Beatmung eines humanen Patienten 12.

Die Beatmungsvorrichtung 10 weist ein Gehäuse 14 auf, in welchem eine Ansaugöffnung 15 ausgebildet und - von außen wegen des blickdichten Gehäusematerials nicht erkennbar - eine Strömungsveränderungsvorrichtung 16 sowie eine Steuereinrichtung 18 aufgenommen sind. Die Ansaugöffnung 15 gestattet der Strömungsveränderungsvorrichtung 16, Umgebungsluft aus der Außenumgebung U der Beatmungsvorrichtung anzusaugen und nach an sich bekannter Reinigung durch Filter als Atemgas dem Patienten 12 zuzuführen. Die Ansaugöffnung 15 ist daher eine Atemgas-Quellenanordnung im Sinne der vorliegenden Anmeldung.

In der Ansaugöffnung 15 kann sich ein Umgebungstemperatursensor 17 befinden, welcher die Temperatur der Luft der Umgebung U misst und an die Steuervorrichtung 18 überträgt.

Die Strömungsveränderungsvorrichtung 16 ist in an sich bekannter Weise aufgebaut und kann eine Pumpe, einen Verdichter, ein Gebläse, einen Druckbehälter, ein Reduzierventil und dergleichen aufweisen. Weiter weist die Beatmungsvorrichtung 10 in an sich bekannter Weise ein Inspirationsventil 20 und ein Exspirationsventil 22 auf.

Die Steuereinrichtung 18 ist üblicherweise als Computer oder Mikroprozessor realisiert. Sie umfasst einen in Figur 1 mit 19 bezeichneten Datenspeicher, um für den Betrieb der Beatmungsvorrichtung 10 notwendige Daten speichern und erforderlichenfalls abrufen zu können. Der Datenspeicher 19 kann bei Netzwerkbetrieb auch außerhalb des Gehäuses 14 gelegen und durch eine Datenübertragungsverbindung mit der Steuereinrichtung 18 verbunden sein. Die Datenübertragungsverbindung kann durch eine Kabel- oder eine Funkstrecke gebildet sein. Um jedoch zu verhindern, dass Störungen der Datenübertragungsverbindung sich auf den Betrieb der Beatmungsvorrichtung 10 auswirken können, ist der Datenspeicher 19 bevorzugt in die Steuereinrichtung 18 integriert oder wenigstens im selben Gehäuse 14 wie diese aufgenommen.

Zur Eingabe von Daten in die Beatmungsvorrichtung 10 bzw. genauer in die Steuereinrichtung 18 kann die Beatmungsvorrichtung 10 eine Eingabevorrichtung 24 aufweisen, welche in dem in Figur 1 dargestellten Beispiel durch eine Tastatur repräsentiert ist. Wie weiter unten noch erläutert werden wird, ist die Tastatur nicht notwendigerweise der einzige Dateneingang der Steuereinrichtung 18. Tatsächlich kann die Steuereinrichtung 18 zusätzlich oder alternativ zur Tastatur Daten über verschiedene Dateneingänge erhalten, etwa über eine Netzwerkleitung, eine Funkstrecke oder über Sensoranschlüsse 26.

Zur Ausgabe von Daten an den behandelnden Therapeuten kann die Beatmungsvorrichtung 10 ein Ausgabegerät 28 aufweisen, im dargestellten Beispiel einen Bildschirm.

Zur künstlichen Beatmung ist der Patient 12 mit der Beatmungsvorrichtung 10, genauer mit der Strömungsveränderungsvorrichtung 16 im Gehäuse 14, über eine Atemgasleitungsanordnung 30 verbunden. Der Patient 12 ist hierfür mittels eines Endotrachealtubus als einer Patientenschnittstelle 31 intubiert. Ein proximales Längsende 31a der Patientenschnittstelle 31 gibt den inspiratorischen Atemgasfluss AF in die Lunge des Patienten 12 ab. Durch das proximale Längsende 31a strömt auch der exspiratorische Atemgasstrom EF in die Atemgasleitungsanordnung 30 ein.

Ein distales Längsende 31b der Patientenschnittstelle 31 ist zur Verbindung mit der Atemgasleitungsanordnung 30 ausgebildet. Ab dem Ort 31c in Inspirationsrichtung stromabwärts bis zum proximalen Längsende 31a ist die Patientenschnittstelle vom Körper des Patienten 12 umgeben. Dies bedeutet im Umkehrschluss, dass die Patientenschnittstelle 31 von seinem distalen Längsende 31b bis zum Ort 31c der Außenumgebung U ausgesetzt ist und mit dieser in, überwiegend konvektiver, Wärmeübertragungsverbindung steht.

Die Atemgasleitungsanordnung 30 weist einen Inspirationsschlauch 32 auf, über welchen frisches Atemgas von der Strömungsveränderungsvorrichtung 16 in die Lunge des Patienten 12 geleitet werden kann. Der Inspirationsschlauch 32 kann unterbrochen sein und einen ersten Inspirationsschlauch 34 und einen zweiten Inspirationsschlauch 36 aufweisen, zwischen welchen eine Befeuchtungsvorrichtung 38 zur gezielten Befeuchtung und gegebenenfalls auch Temperierung des dem Patienten 12 zugeführten inspiratorischen Atemgases vorgesehen sein kann. Die Befeuchtungsvorrichtung 38 kann mit einem externen Flüssigkeitsvorrat 40 verbunden sein, über den Wasser zur Befeuchtung oder auch ein Medikament, etwa zur Entzündungshemmung oder zur Erweiterung der Atemwege, der Befeuchtungsvorrichtung 38 zugeführt werden kann. Bei einem Einsatz der vorliegenden Beatmungsvorrichtung 10 als Anästhesie-Beatmungsvorrichtung können auf diese Weise volatile Anästhetika kontrolliert über die Beatmungsvorrichtung 10 an den Patienten 12 abgegeben werden. Die Befeuchtungsvorrichtung 38 sorgt dafür, dass das frische Atemgas dem Patienten 12 mit einer vorbestimmten Feuchtigkeit, gegebenenfalls unter Zugabe eines Medikamenten-Aerosols, und mit einer vorbestimmten Temperatur zugeführt wird.

Der zweite Inspirationsschlauch 36 ist im vorliegenden Beispiel durch eine Leitungsheizvorrichtung 37 elektrisch beheizbar. Die Leitungsheizvorrichtung 37 ist durch die Steuervorrichtung 18 zum Betrieb ansteuerbar. Abweichend vom Gesagten kann auch der erste Inspirationsschlauch 34 beheizbar sein oder/und kann der wenigstens eine Schlauch 34 oder/und 36 durch eine andere als eine elektrische Leitungsheizvorrichtung 37 beheizbar sein, etwa durch Umspülung mit einem Wärmetauschmedium.

Die Atemgasleitungsanordnung 30 weist neben dem bereits erwähnten Inspirationsventil 20 und Exspirationsventil 22 weiter einen Exspirationsschlauch 42 auf, über welchen verstoffwechseltes Atemgas als exspiratorischer Atemgasfluss EF aus der Lunge des Patienten 12 in die Außenumgebung U abgeblasen wird.

Am distalen Längsende 30b der Atemgasleitungsanordnung 30 sind der Inspirationsschlauch 32 mit dem Inspirationsventil 20 und der Exspirationsschlauch 42 mit dem Exspirationsventil 22 gekoppelt. Von den beiden Ventilen ist vorzugsweise jeweils nur eines gleichzeitig zum Durchlass einer Gasströmung geöffnet. Die Betätigungssteuerung der Ventile 20 und 22 erfolgt ebenfalls durch die Steuereinrichtung 18.

Während eines Beatmungszyklus ist zunächst für die Dauer der Inspirationsphase das Exspirationsventil 22 geschlossen und das Inspirationsventil 20 geöffnet, sodass frisches inspiratorisches Atemgas vom Gehäuse 14 zum Patienten 12 geleitet werden kann. Eine Strömung des frischen Atemgases wird durch gezielte Druckerhöhung des Atemgases durch die Strömungsveränderungsvorrichtung 16 bewirkt. Aufgrund der Druckerhöhung strömt das frische Atemgas in die Lunge des Patienten 12 und expandiert dort den lungennahen Körperbereich, also insbesondere den Brustkorb, gegen die individuelle Elastizität der lungennahen Körperteile. Hierdurch steigt auch der Gasdruck im Inneren der Lunge des Patienten 12 an.

Am Ende der Inspirationsphase werden das Inspirationsventil 20 geschlossen und das Exspirationsventil 22 geöffnet. Es beginnt die Exspirationsphase. Aufgrund des bis zum Ende der Inspirationsphase erhöhten Gasdrucks des in der Lunge des Patienten 12 befindlichen Atemgases strömt dieses nach dem Öffnen des Exspirationsventils 22 in die Außenumgebung U, wobei sich mit fortschreitender Strömungsdauer der Gasdruck in der Lunge des Patienten 12 verringert. Erreicht der Gasdruck in der Lunge 12 einen an der Beatmungsvorrichtung 10 eingestellten positiven end-exspiratorischen Druck (PEEP), also einen geringfügig höheren Druck als den Atmosphärendruck, wird die Exspirationsphase mit dem Schließen des Exspirationsventils 22 beendet und es schließt sich ein weiterer Beatmungszyklus an.

Während der Inspirationsphase wird dem Patienten 12 das sogenannte Beatmungs-Tidalvolumen zugeführt, also das Atemgas-Volumen pro Atemhub. Das Beatmungs-Tidalvolumen multipliziert mit der Anzahl an Beatmungszyklen pro Minute, also multipliziert mit der Beatmungsfrequenz, ergibt das Minutenvolumen der vorliegend durchgeführten künstlichen Beatmung.

Bevorzugt ist die Beatmungsvorrichtung 10, insbesondere die Steuereinrichtung 18, dazu ausgebildet, Beatmungs-Betriebsparameter, die den Beatmungsbetrieb der Beatmungsvorrichtung 10 kennzeichnen, während des Beatmungsbetriebs wiederholt zu aktualisieren bzw. zu ermitteln, um sicherzustellen, dass der Beatmungsbetrieb zu jedem Zeitpunkt möglichst optimal auf den jeweils zu beatmenden Patienten 12 abgestimmt ist. Besonders vorteilhaft erfolgt die Bestimmung eines oder mehrerer Beatmungs-Betriebsparameter mit der Beatmungsfrequenz, sodass für jeden Beatmungszyklus aktuelle und damit optimal an den Patienten 12 angepasste Beatmungs-Betriebsparameter bereitgestellt werden können.

Hierzu kann die Beatmungsvorrichtung 10 mit einem oder mehreren Sensoren datenübertragungsmäßig verbunden sein, welche den Zustand des Patienten oder/und den Betrieb der Beatmungsvorrichtung 10 überwachen. Lediglich beispielhaft für eine Reihe möglicher Sensoren sei in Figur 1 ein proximaler Flusssensor 44 genannt, welcher den in der Atemgasleitungsanordnung 30 herrschenden Atemgasfluss, und zwar sowohl den inspiratorischen Atemgasfluss AF wie auch den exspiratorischen Atemgasfluss EF, betragsmäßig erfasst. Der vorzugsweise als Differenzdrucksensor ausgebildete proximale Flusssensor 44 kann mittels einer Sensorleitungsanordnung 46 mit den Dateneingängen 26 der Steuereinrichtung 18 gekoppelt sein. Die Sensorleitungsanordnung 46 kann, muss jedoch nicht, elektrische Signalübertragungsleitungen umfassen. Sie kann ebenso Schlauchleitungen aufweisen, die den in Strömungsrichtung beiderseits des Flusssensors 44 herrschenden Gasdruck an die Dateneingänge 26 übertragen, wo diese von Drucksensoren 27 quantifiziert werden.

Genauer weist die Atemgasleitungsanordnung 30 im bevorzugten Ausführungsbeispiel an ihrem proximalen Längsendbereich 30a einen gesondert ausgebildeten Y-Leitungsabschnitt 47 auf, welcher an seinem distalen Endbereich mit dem zweiten Inspirationsschlauch 36 und dem Exspirationsschlauch 42 verbunden ist und welcher an seinem proximalen Endbereich mit dem proximale Flusssensor 44 verbunden ist.

Der proximale Flusssensor 44 weist an seinem proximalen Endbereich eine Kopplungsformation 44a auf, mit welcher die Patientenschnittstelle 31, die anstelle eines Tubus auch eine Maske sein könnte, mit dem proximalen Flusssensor 44 und folglich mit der Atemgasleitungsanordnung 30 koppelbar ist.

Der zweite Inspirationsschlauch 36 kann an seinem proximalen Längsendbereich einen proximalen Temperatursensor 48 aufweisen, welcher die Temperatur des Atemgasflusses AF im zweiten Inspirationsschlauch 36 möglichst nah am Patienten 12 misst und an die Steuervorrichtung 18 überträgt.

Lediglich der Vollständigkeit halber sei darauf hingewiesen, dass die erfindungsgemäße Beatmungsvorrichtung 10 als mobile Beatmungsvorrichtung 10 auf einem rollbaren Gestell 50 aufgenommen sein kann.

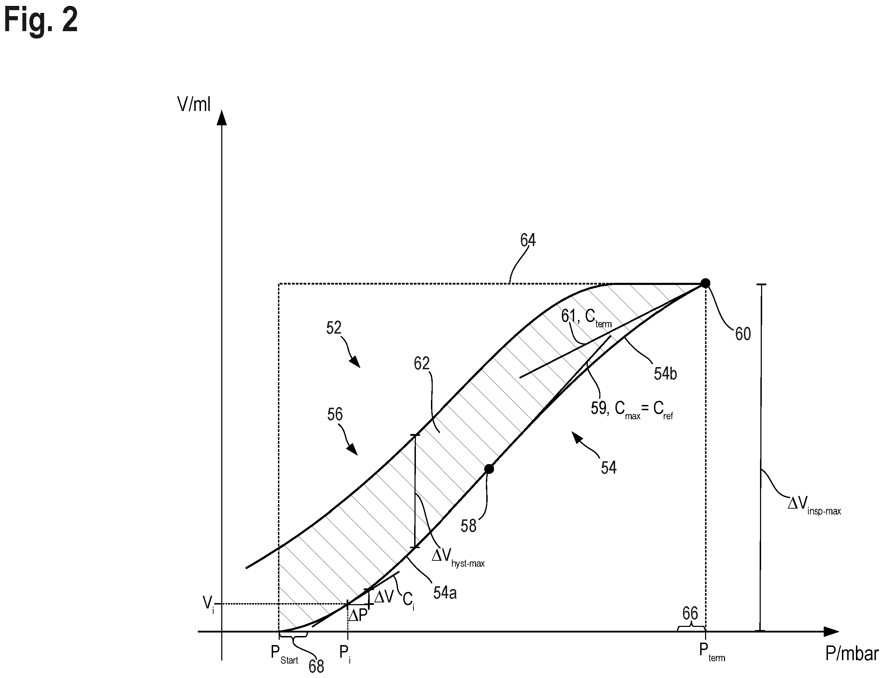

In Figur 2 ist eine P-V-Kurve grobschematisch dargestellt und mit 52 bezeichnet, durch ein P/V-Manöver erhalten wurde, dass mit der Beatmungsvorrichtung 10 von Figur 1 durchgeführt wurde. Das P/V-Manöver der vorliegenden Anmeldung kann ein sich vom übrigen Beatmungsbetrieb unterscheidendes, einmaliges Manöver sein. Eine Folge von P/V-Manövern der vorliegenden Anmeldung kann jedoch auch eine Abfolge von Atemhüben zur Verabreichung des Beatmungs-Tidalvolumens und somit Teil der medizinisch indizierten regulären künstlichen Beatmung des Patienten 12 sein.

Die Abszisse des Koordinatensystems gibt in Pfeilrichtung steigende Drücke des Atemgases an, die Ordinate des Koordinatensystems gibt in Pfeilrichtung steigende Manöver-Atemgasvolumina an. Die Darstellung der P-V-Kurve in dem Koordinatensystem ist lediglich beispielhaft und grobschematisch. Der Kreuzungspunkt des Koordinatensystems ist nicht notwendigerweise der Koordinatenursprung bei einem Druck von 0 mbar und einem Manöver-Atemgasvolumens von 0 ml.

Das P/V-Manöver beginnt bei einem Start-Atemgasdruck P_{Start} mit einer Inspirationsphase, d. h. der Start-Atemgasdruck P_{Start} ist hoch genug, um inspiratorisches Atemgas in die Lunge des Patienten 12 einzuleiten.

Ab Beginn der Inspirationsphase erfasst die Steuervorrichtung 18 über den Flusssensor 44 und die zugehörigen Drucksensoren 27 den inspiratorischen Atemgasfluss AF und dessen Druck in der Atemgasleitungsanordnung 30 quantitativ und bildet so Wertepaare aus einem inspiratorischen Atemgasdruck und einem dem Druck zugeordneten, dem Patienten 12 verabreichten Manöver-Atemgasvolumen. Das verabreichte Manöver-Atemgasvolumen entspricht dem Integral des inspiratorischen Atemgasflusses vom Zeitpunkt des Beginns der Inspirationsphase bis zum Erfassungszeitpunkt. So ermittelt die Steuervorrichtung 18 die in Figur 2 dargestellte P-V-Kurve. Zunächst wird der inspiratorische Ast 54 der P-V-Kurve 52 ermittelt. Nach Abschluss der Inspirationsphase wird der exspiratorische Ast 56 ermittelt, indem man das zunächst dem Patienten 12 zugeführte Atemgas passiv, d. h. alleine unter dem Druck des im Patienten 12 vorhandenen Atemgases, aus dem Patienten 12 in die Umgebung U entweichen lässt.

Von Beginn der Inspirationsphase an ermittelt die Steuervorrichtung 18 aus dem Unterschied zwischen aufeinanderfolgenden inspiratorischen Atemgasdrücken und dem Unterschied der diesen Atemgasdrücken zugeordneten Manöver-Atemgasvolumina die einem inspiratorischen Atemgasdruck, welcher betragsmäßig - einschließlich der Bereichsgrenzen - in dem zur Ermittlung des Atemgasdruck-Unterschieds herangezogenen Bereich von inspiratorischen Atemgasdrücken liegt, zugeordnete Lungen-Compliance Ci als Funktion ebenfalls des Atemgasdrucks. Dies ist Figur 2 beispielhaft durch das Steigungsdreieck ΔV/ΔP bei dem Atemgasdruck Pᵢ angedeutet. Bezogen auf P-V-Kurve von Figur 2 bedeutet dies, dass die Lungen-Compliance Ci während der Inspirationsphase die erste Ableitung der P-V-Kurve nach dem Atemgasdruck ist. Somit sind grundsätzlich unterschiedliche Verfahren zur Ermittlung der einem jeweiligen Atemgasdruck zugeordneten Lungen-Compliance Ci verfügbar und nutzbar.

Die Steuervorrichtung 18 speichert die so ermittelten Wertepaare aus Lungen-Compliance Ci und zugeordnetem Atemgasdruck Pᵢ im Datenspeicher 19 ab und ermittelt aus den abgespeicherten Werten den größten auftretenden Wert Cₘₐₓ der Lungen-Compliance. Dabei kann man sich die Tatsache zunutze machen, dass Lungen zu Beginn einer Inspirationsphase und am Ende einer Inspirationsphase jeweils betragsmäßig niedrigere Lungen-Compliancewerte Ci aufweisen als in einem mittleren Bereich der Inspirationsphase. Ist folglich ein betragsmäßig größter Wert Cₘₐₓ von Lungen-Compliances Ci erreicht und weisen nachfolgend bei höheren inspiratorischen Atemgasdrücken ermittelte Lungen-Compliances Ci betragsmäßig geringere Werte auf, so ist mit dem betragsmäßig größten Wert auch der für die gesamte Inspirationsphase absolut größte Wert der Lungen-Compliance Cₘₐₓ erkannt.

Die P-V-Kurve kann mit üblichen Glättungsverfahren geglättet werden, um Rauschanteile auszufinden und umso eine stabilere Ermittlung der Lungen-Compliance zu erhalten.

Die größte Lungen-Compliance Cₘₐₓ herrscht in Figur 2 am Punkt 58. Die dort herrschende größte Steigung ist durch die Tangente 59 an den inspiratorischen Ast 54 der P-V-Kurve 52 im Punkt 58 angezeigt.

Der so ermittelte Wert der größten Lungen-Compliance Cₘₐₓ wird von der Steuervorrichtung 18 als Bezugs-Compliancewert C_{ref} ausgewählt. Nach der Auswahl des Bezugs-Compliancewerts C_{ref} errechnet die Steuervorrichtung 18 automatisch einen Abbruch-Compliancewert Cₜₑᵣₘ, bei welchem die Inspirationsphase des P/V-Manövers abgebrochen wird. Im vorliegenden Ausführungsbeispiel multipliziert die Steuervorrichtung 18 den Bezugs-Compliancewert C_{ref} mit einem vorbestimmten Faktor, der kleiner als 1 ist, beispielsweise mit 0,9, um so den Abbruch-Compliancewert Cₜₑᵣₘ zu errechnen.

Fortan vergleicht die Steuervorrichtung 18 für steigende Atemgasdrücke die jeweils ermittelte Lungen-Compliance Ci mit dem Abbruch-Compliancewert Cₜₑᵣₘ und beendet die Inspirationsphase dann, wenn sie erkennt, dass die momentane Lungen-Compliance Ci den Abbruch-Compliancewert Cₜₑᵣₘ erreicht oder überschritten hat.

Dies ist in Figur 2 bei Punkt 60 der Fall, wo wiederum durch die Tangente 61 an den inspiratorischen Ast 54 der P-V-Kurve 52 in Punkt 60 die Lungen-Compliance Cₜₑᵣₘ sichtbar gemacht ist.

Alternativ oder zusätzlich kann die Steuervorrichtung 18 aus dem Verlauf der Manöver-Atemgasvolumina als Funktion des Atemgasdrucks den Punkt 58 als Wendepunkt zwischen einem konkaven Abschnitt 54a bei betragsmäßig niedrigeren Atemgasdrücken und einem konvexen Abschnitt 54b bei betragsmäßig höheren Atemgasdrücken als Ort der betragsmäßig größten Lungen-Compliance Cₘₐₓ und damit als Bezugs-Compliancewert C_{ref} ermitteln.

Durch den Abbruch der Inspirationsphase im Punkt 60 kann die Lunge des Patienten 12 automatisiert vor einem Barotrauma oder vor einer sonstigen Schädigung durch für den jeweiligen Patienten 12 zu hohen Atemgasdruck in der Lunge geschützt werden. Das Abbruchkriterium in Gestalt des Abbruch-Compliancewerts Cₜₑᵣₘ wird dabei während des dadurch abzubrechenden P/V-Manövers ermittelt und sogleich angewendet.

In der anschließenden Exspirationsphase, repräsentiert durch den Ast 56 der P-V-Kurve 52, strömt exspiratorisches Atemgas aus der Patientenlunge gemäß den obigen Erläuterungen zu Figur 1 in die Umgebung U. Dabei stellt sich zwischen dem exspiratorischen Ast 56 und dem inspiratorischen Ast 54 die für P/V-Manöver bekannte Hysterese ein.

Wenngleich bereits bei dem P/V-Manöver selbst Lungengewebe für kurze Zeit rekrutiert werden kann, was beispielsweise die Ursache dafür sein kann, dass während der Exspirationsphase beim Start-Atemgasdruck P_{Start} ein höheres Manöver-Atemgasvolumen im Patienten 12 vorhanden ist als während der Inspirationsphase, ist das Hystereseverhalten der Lunge des Patienten 12 während des P/V-Manövers ein zuverlässiger Indikator für eine Aussage über die Erfolgsaussichten von medizinisch an sich bekannten Recruitment-Verfahren an der Patientenlunge, um Lungengewebe für einen Gasaustausch zu rekrutieren.

Dabei ist der rechtzeitige automatisierte Abbruch des P/V-Manövers vor einem Erreichen zu hoher inspiratorischer Atemgasdrücke auch für die Erhöhung der Aussagekraft der erhaltenen P-V-Kurve über die Erfolgsaussichten zeitnah zukünftiger Recruitment-Verfahren zur Rekrutierung von Lungengewebe des Patienten 12 vorteilhaft. Das Vorantreiben des P/V-Manövers zu vorbestimmten hohen Enddrücken, bei welchen die Lunge des Patienten bereits mehr oder weniger stark überdehnt wird, würde weniger aussagekräftige P-V-Kurven liefern.

Beispielsweise kann die Steuervorrichtung 18 dazu ausgebildet sein den Flächeninhalt der Hysteresefläche 62 zu quantifizieren, welche die beiden Äste 54 und 56 zwischen dem Start-Atemgasdruck P_{Start} und dem am Abbruchpunkt 60 herrschenden Abbruch-Atemgasdruck Pₜₑᵣₘ zwischen sich einschließen. Weiter kann die Steuervorrichtung 18 dazu ausgebildet sein den Flächeninhalt eines Rechtecks 64 zu berechnen, dessen eine Ecke bei dem Wertepaar aus Start-Atemgasdruck P_{Start} und dem dem Start-Atemgasdruck P_{Start} zugeordneten inspiratorischen Manöver-Atemgasvolumen gelegen ist und dessen diagonal gegenüberliegende Ecke beim Abbruch-Atemgasdruck Pₜₑᵣₘ und dem dem Abbruch-Atemgasdruck Pₜₑᵣₘ zugeordneten Manöver-Atemgasvolumen gelegen ist. Da am Punkt 60, wo der Abbruch-Atemgasdruck Pₜₑᵣₘ herrscht, das inspiratorische und das exspiratorische Manöver-Atemgasvolumen in der Regel gleich groß sind, kommt es hier auf die Wahl des Manöver-Atemgasvolumenwerts aus inspiratorischen und exspiratorischen Manöver-Atemgasvolumens nicht an.

Die Steuervorrichtung 18 kann weiter ausgebildet sein, einen Hysterese-Quotientenwert aus dem Flächeninhalt der Hysteresefläche und dem Flächeninhalt der Rechteckfläche zu berechnen und mit einem vorbestimmten ersten Schwellenwert zu vergleichen. Dann, wenn der Hysterese-Quotientenwert größer als der vorbestimmte ersten Schwellenwert ist, gibt die Steuervorrichtung 18 über das Ausgabegerät 28 eine Ausgabe aus, welche anzeigt, dass ein Recruitment-Verfahren an der derzeit beatmenden Lunge überwiegend Aussicht auf Rekrutierungserfolg hat.

Der Start-Atemgasdruck P_{Start} kann der für den Patienten eingestellte PEEP sein oder kann bis zu etwa dem 1,6 fachen des eingestellten PEEP sein. Bevorzugt liegt der Start-Atemgasdruck des P/V-Manövers - unabhängig vom vorliegend beschriebenen Ausführungsbeispiel - in einem Druckbereich von 5 bis 8 mbar, vorzugsweise von 7 bis 8 mbar.

Alternativ oder zusätzlich kann die Steuervorrichtung 18 dazu ausgebildet sein, den betragsmäßig größten Unterschied zwischen dem exspiratorischen Ast 56 und dem inspiratorischen Ast 54 zu ermitteln. Dieser ist in Figur 2 als ΔV_{hyst-max} bezeichnet.

Weiter kann die Steuervorrichtung 18 dazu ausgebildet sein den größten Volumenunterschied an inspiratorischem Manöver-Atemgasvolumen zu ermitteln, das ist in der Regel die Differenz der Volumenkoordinaten des inspiratorischen Astes 54 am Abbruch-Atemgasdruck Pₜₑᵣₘ einerseits und am Start-Atemgasdruck P_{Start} andererseits. Dieser Unterschied ist in Figur 2 als ΔVᵢₙₛₚ₋ₘₐₓ bezeichnet.

Die Steuervorrichtung 18 kann weiter dazu ausgebildet sein, einen Volumen-Quotientenwert aus dem ermittelten größten Volumenunterschied zwischen dem exspiratorischen Ast 56 und dem inspiratorischen Ast 54 und dem größten Volumenunterschied ΔVᵢₙₛₚ₋ₘₐₓ an inspiratorischen Manöver-Atemgasvolumen zu berechnen und mit einem vorbestimmten zweiten Schwellenwert zu vergleichen. Dann wenn der Volumen-Quotientenwert größer ist als der vorbestimmte zweite Schwellenwert, gibt die Steuervorrichtung 18 wiederum über das Ausgabegerät 28 eine Ausgabe aus, wonach ein Recruitment-Verfahren, durchgeführt an der aktuell beatmeten Patientenlunge, überwiegend Aussicht auf Erfolg hat.

Die in Figur 2 rechte vertikale Seite des Rechtecks 64, ebenso wie der größte inspiratorische Volumenunterschied ΔVᵢₙₛₚ₋ₘₐₓ liegen lediglich besonders bevorzugt beim Druckwert des Abbruch-Atemgasdrucks Pₜₑᵣₘ. Die Aussagekraft der zuvor beschriebenen Kriterien für die Beurteilung der Erfolgsaussichten eines Recruitment-Verfahren an der Patientenlunge ist immer noch ausreichend zuverlässig, wenn die rechte vertikale Seite des Rechtecks 64 oder/und der größte inspiratorische Volumenunterschied ΔVᵢₙₛₚ₋ₘₐₓ in einem oberen Endbereich 66 des während des P/V-Manövers durchlaufenden Atemgasdruckbereichs liegen, welcher von 95 % bis 100 % des Abbruch-Atemgasdrucks Pₜₑᵣₘ reicht.

Ebenso kann die linke vertikale Seite des Rechtecks 64 in einem unteren Endbereich 68 des während des P/V-Manövers durchlaufenden Atemgasdruckbereichs liegen, welcher ausgehend vom Start-Atemgasdruck P_{Start} bis zum 1,05-Fachen des Start-Atemgasdrucks P_{Start} reicht. Die linke vertikale Seite des Rechtecks 64 muss daher nicht unmittelbar am Start-Atemgasdruck P_{Start} gelegen sein, wenngleich dies bevorzugt ist.

Auf diese Weise können Patienten ohne aufwändige computertomografische Verfahren hinsichtlich der Erfolgsaussichten von an ihnen ausgeführten Recruitment-Verfahren zu Rekrutierung von Lungengewebe unmittelbar am Ort ihrer Beatmung beurteilt werden.

## Patentansprüche

1. Beatmungsvorrichtung (10) zur künstlichen Beatmung eines Patienten (12), umfassend:
- eine Atemgas-Quellenanordnung (15), welche ein inspiratorisches Atemgas zur künstlichen Beatmung des Patienten (12) bereitstellt,
- eine Strömungsveränderungsvorrichtung (16), welche dazu ausgebildet ist, einen inspiratorischen Atemgasfluss (AF) zu erzeugen und betragsmäßig zu verändern,
- eine Atemgasleitungsanordnung (30) mit einem im Betrieb dem Patienten (12) näher gelegenen proximalen Längsende (30a) und mit einem im Betrieb vom Patienten weiter entfernt gelegenen distalen Längsende (30b), um den inspiratorischen Atemgasfluss (AF) von der Atemgas-Quellenanordnung (15, 62) zum Patienten (12) hin zu fördern,
- eine Flusssensoranordnung (44), welcher dazu ausgebildet ist, den inspiratorischen Atemgasfluss (AF) ebenso wie einen exspiratorischen Atemgasfluss (EF) betragsmäßig zu erfassen,
- eine Drucksensoranordnung (27), welche dazu ausgebildet ist, einen Druck des inspiratorischen Atemgases ebenso wie des exspiratorischen Atemgases in der Atemgasleitungsanordnung (30) zu erfassen,
- eine Steuervorrichtung (18) mit einem Datenspeicher (19), wobei die Steuervorrichtung (18) signalübertragungsmäßig mit dem Datenspeicher (19), mit der Flusssensoranordnung (44) und mit der Drucksensoranordnung (27) verbunden ist und welche dazu ausgebildet ist, die Betriebsleistung der Strömungsveränderungsvorrichtung (16) zur Veränderung des inspiratorischen Atemgasflusses (AF) zu steuern,
wobei die Steuervorrichtung (18) dazu ausgebildet ist, die Strömungsveränderungsvorrichtung (16) zur Durchführung eines P/V-Manövers anzusteuern, bei welchem einem Patienten (12) in einer Inspirationsphase Atemgas unter Erhöhung des Atemgasdrucks zugeführt wird, welches in einer Exspirationsphase nach Abschluss der Druckerhöhung passiv aus dem Patienten ausströmt, wobei sowohl während der Inspirationsphase als auch während der Exspirationsphase für eine Vielzahl von Atemgasdrücken das jeweils aufgrund des P/V-Manövers im Patienten vorhandene Manöver-Atemgasvolumen in Zuordnung zum herrschenden Atemgasdruck ermittelt wird,
**dadurch gekennzeichnet, dass** die Steuervorrichtung (18) dazu ausgebildet ist,
- während der Inspirationsphase auf Grundlage von Signalen der Flusssensoranordnung (44) und der Drucksensoranordnung (27) eine Folge von Compliancewerten zu ermitteln, welche jeweils eine Lungen-Compliance der Lunge des Patienten (12) repräsentieren,
- nach Maßgabe der Folge von Compliancewerten (Ci) einen Bezugs-Compliancewert (C_{ref}) zu ermitteln,
- als Abbruchkriterium für die Inspirationsphase ausgehend von dem Bezugs-Compliancewert (C_{ref}) einen vom Bezugs-Compliancewert (C_{ref}) betragsmäßig verschiedenen Abbruch-Compliancewert (Cₜₑᵣₘ) als Schwellenwert zu bestimmen, und
- die Inspirationsphase dann abzubrechen, wenn der Abbruch-Compliancewert (Cₜₑᵣₘ) erreicht oder durchschritten wird.

2. Beatmungsvorrichtung (10) nach Anspruch 1,
**dadurch gekennzeichnet, dass** die Steuervorrichtung (18) dazu ausgebildet ist, die Folge von Compliacewerten (Ci) zu berechnen aus
i) einem Quotienten aus einem einem Atemgasdruck zugeordneten Volumenänderungswert (ΔV) und einem demselben Atemgasdruck zugeordneten Druckänderungswert (ΔP), wobei der Volumenänderungswert (ΔV) eine zeitliche Veränderung des Manöver-Atemgasvolumens repräsentiert und wobei der Druckänderungswert (ΔV) eine zeitliche Veränderung des Atemgasdrucks repräsentiert,
oder/und
ii) einem Quotienten aus einem einem Atemgasdruck zugeordneten Flusswert und dem demselben Atemgasdruck zugeordneten Druckänderungswert (ΔP), wobei der Flusswert den inspiratorischen Atemgasfluss (AF) repräsentiert.

3. Beatmungsvorrichtung (10) nach Anspruch 1 oder 2,
**dadurch gekennzeichnet, dass** die Steuervorrichtung (18) dazu ausgebildet ist, aus einer Folge von betragsmäßig zunächst größer und anschließend kleiner werdenden Compliancewerten (Ci) den betragsmäßig größten Compliancewert (Cₘₐₓ) als Bezugs-Compliancewert (C_{ref}) auszuwählen.

4. Beatmungsvorrichtung (10) nach einem der vorhergehenden Ansprüche, **dadurch gekennzeichnet, dass** die Steuervorrichtung (18) dazu ausgebildet ist, aus einer Folge von Wertepaaren aus inspiratorischem Atemgasdruck und dem jeweiligen inspiratorischen Atemgasdruck zugeordneten Manöver-Atemgasvolumen einen Wendepunkt (58) zwischen mit unterschiedlichem Krümmungssinn gekrümmten Abschnitten (54a, 54b) eines die Folge von Wertepaaren repräsentierenden Graphen (54) zu ermitteln und den dem Atemgasdruck am Wendepunkt (58) zugeordneten Compliancewert als Bezugs-Compliancewert (C_{ref}) auszuwählen.

5. Beatmungsvorrichtung (10) nach einem der vorhergehenden Ansprüche, **dadurch gekennzeichnet, dass** die Steuervorrichtung (18) dazu ausgebildet ist, den Abbruch-Compliancewert (Cₜₑᵣₘ) durch Multiplikation des Bezugs-Compliancewerts (C_{ref}) mit einem vorbestimmten Faktor oder durch Addition des Bezugs-Compliancewerts (C_{ref}) mit einem vorbestimmten Summanden zu berechnen.

6. Beatmungsvorrichtung (10) nach Anspruch 5,
**dadurch gekennzeichnet, dass** der Abbruch-Compliancewert (Cₜₑᵣₘ) 75 % bis 95 %, vorzugsweise 80 % bis 92,5%, besonders bevorzugt 85 % bis 91 % des Bezugs-Compliancewerts (C_{ref}) beträgt.

7. Beatmungsvorrichtung (10) nach einem der vorhergehenden Ansprüche, **dadurch gekennzeichnet, dass** die Steuervorrichtung (18) dazu ausgebildet ist, einen Volumen-Quotientenwert zu berechnen aus dem betragsmäßig größten während des P/V-Manövers für einen Atemgasdruck auftretenden Unterschied (ΔV_{hyst-max}) zwischen dem exspiratorischen (56) und dem inspiratorischen Manöver-Atemgasvolumen (54) und einem Unterschiedswert (ΔVᵢₙₛₚ₋ₘₐₓ) zwischen einem Manöver-Atemgasvolumenwert in einem oberen Endbereich (66) und einem Manöver-Atemgasvolumenwert in einem unteren Endbereich (68) des während des P/V-Manövers durchlaufenden Atemgasdruckbereichs, wobei der untere Endbereich (68) einen Start-Atemgasdruck (P_{Start}) enthält, und bis zum 1,05-Fachen des Start-Atemgasdruck (P_{Start}) reicht, und wobei der obere Endbereich (66) einen Abbruch-Atemgasdruck (Pₜₑᵣₘ) enthält, dem der Abbruch-Compliancewert (Cₜₑᵣₘ) zugeordnet ist, und bei 95 % des Abbruch-Atemgasdrucks (Pₜₑᵣₘ) beginnt.

8. Beatmungsvorrichtung (10) nach Anspruch 7,
**dadurch gekennzeichnet, dass** die Steuervorrichtung (18) dazu ausgebildet ist, dann, wenn der Volumen-Quotientenwert einen vorbestimmten ersten Schwellenwert übersteigt, eine Ausgabe zu erzeugen, welche anzeigt, dass ein Recruitment-Verfahren zu Rekrutierung der Patientenlunge überwiegend Aussicht auf Erfolg hat.

9. Beatmungsvorrichtung (10) nach einem der vorhergehenden Ansprüche, **dadurch gekennzeichnet, dass** die Steuervorrichtung (18) dazu ausgebildet ist, einen Hysterese-Quotientenwert zu berechnen aus dem Flächeninhalt der Hysterese-Fläche (62), welche die Graphen der exspiratorischen (56) und der inspiratorischen Manöver-Atemgasvolumina (54) als Funktionen des Atemgasdrucks zwischen einem Start-Atemgasdruck (P_{Start}), bei welchem das P/V-Manöver beginnt, und einen Abbruch-Atemgasdruck (Pₜₑᵣₘ), dem der Abbruch-Compliancewert (Cₜₑᵣₘ) zugeordnet ist, und dem Flächeninhalt eines die Hysterese-Fläche umschreibenden Rechtecks (64), dessen eine Ecke bestimmt ist durch einen unteren Atemgasdruckwert in einem unteren Endbereich (68) des während des P/V-Manövers durchlaufenden Atemgasdruckbereichs und durch den dem unteren Atemgasdruckwert zugeordneten Atemgasvolumenwert, wobei der untere Endbereich (68) den Start-Atemgasdruck (P_{Start}) enthält, und bis zum 1,05-Fachen des Start-Atemgasdruck (P_{Start}) reicht, und dessen diagonal gegenüberliegende Ecke bestimmt ist durch einen oberen Atemgasdruckwert in einem oberen Endbereich (66) und durch den dem oberen Atemgasdruckwert zugeordneten Atemgasvolumenwert, wobei der obere Endbereich (66) einen Abbruch-Atemgasdruck (Pₜₑᵣₘ) enthält, dem der Abbruch-Compliancewert (Cₜₑᵣₘ) zugeordnet ist, und bei 95 % des Abbruch-Atemgasdrucks (Pₜₑᵣₘ) beginnt.

10. Beatmungsvorrichtung (10) nach Anspruch 9,
**dadurch gekennzeichnet, dass** die Steuervorrichtung (18) dazu ausgebildet ist, dann, wenn der Hysterese-Quotientenwert einen vorbestimmten zweiten Schwellenwert übersteigt, eine Ausgabe zu erzeugen, welche anzeigt, dass ein Recruitment-Verfahren zu Rekrutierung der Patientenlunge überwiegend Aussicht auf Erfolg hat.

## Claims

1. Ventilation device (10) for artificial ventilation of a patient (12), comprising:
- A respiratory gas source arrangement (15) which provides an inspiratory respiratory gas for artificial ventilation the patient (12),
- A flow modification device (16) which is configured to produce and quantitatively modify an inspiratory respiratory gas flow (AF),
- A respiratory gas line arrangement (30) with a proximal longitudinal end (30a) which during operation lies nearer to the patient (12) and with a distal longitudinal end (30b) which during operation lies further away from the patient, in order to convey the inspiratory respiratory gas flow (AF) from the respiratory gas source arrangement (15, 62) towards the patient (12),
- A flow sensor arrangement (44) which is configured to acquire quantitatively both the inspiratory respiratory gas flow (AF) and an expiratory respiratory gas flow (EF),
- A pressure sensor arrangement (27) which is configured to acquire a pressure both of the inspiratory respiratory gas and of the expiratory respiratory gas in the respiratory gas line arrangement (30),
- A control device (18) with a data memory (19), where the control device (18) is connected for signal transmission with the data memory (19), with the flow sensor arrangement (44), and with the pressure sensor arrangement (27) and which is configured to control the operational output of the flow modification device (16) for modifying the inspiratory respiratory gas flow (AF),
Where the control device (18) is configured to actuate the flow modification device (16) for performing a P/V maneuver in which in an inspiration phase, respiratory gas is supplied to a patient (12) under elevated respiratory gas pressure which in an expiration phase after completion of the pressure elevation flows passively out of the patient, where both during the inspiration phase and during the expiration phase, for a plurality of respiratory gas pressures, the respective maneuver respiratory gas volume present in the patient due to the P/V maneuver is determined in correlation with the prevailing respiratory gas pressure,
**Characterized in that** the control device (18) is configured
- To determine during the inspiration phase, on the basis of signals of the flow sensor arrangement (44) and of the pressure sensor arrangement (27), a sequence of compliance values each of which represents a pulmonary compliance of the lung of the patient (12),
- To determine a reference compliance value (C_{ref}) in accordance with the sequence of compliance values (Ci),
- To determine as termination criterion for the inspiration phase, starting from the reference compliance value (C_{ref}), a termination compliance value (Cₜₑᵣₘ) quantitatively different from the reference compliance value (C_{ref}) as threshold value, and
- To terminate the inspiration phase when the termination compliance value (Cₜₑᵣₘ) is reached or crossed.

2. Ventilation device (10) according to Claim 1,
**Characterized in that** the control device (18) is configured to calculate the sequence of compliance values (Ci) from
i) A ratio of a volume change value (ΔV) associated with a respiratory gas pressure and a pressure change value (ΔP) associated with the same respiratory gas pressure, where the volume change value (ΔV) represents a temporal change in the maneuver respiratory gas volume and where the pressure change value (ΔV) represents a temporal change in the respiratory gas pressure,
and/or
ii) A ratio of a flow value associated with a respiratory gas pressure and the pressure change value (ΔP) associated with the same respiratory gas pressure, where the flow value represents the inspiratory respiratory gas flow (AF).

3. Ventilation device (10) according to Claim 1 or 2,
**Characterized in that** the control device (18) is configured to select, from a sequence of compliance values (Ci) which first become quantitatively larger and subsequently smaller, the quantitatively largest compliance value (Cₘₐₓ) as reference compliance value (C_{ref}).

4. Ventilation device (10) according to one of the preceding Claims, **Characterized in that** the control device (18) is configured to determine, from a sequence of value-pairs of an inspiratory respiratory gas pressure and the maneuver respiratory gas volume associated with the respective inspiratory respiratory gas pressure, an inflection point (58) between sections (54a, 54b) curved in different directions of curvature of a graph (54) representing the sequence of value-pairs, and to select the compliance value associated with the respiratory gas pressure at the inflection point (58) as reference compliance value (C_{ref}).

5. Ventilation device (10) according to one of the preceding Claims, **Characterized in that** the control device (18) is configured to calculate the termination compliance value (Cₜₑᵣₘ) through multiplying the reference compliance value (C_{ref}) by a predetermined factor or through adding the reference compliance value (C_{ref}) to a predetermined summand.

6. Ventilation device (10) according to Claim 5,
**Characterized in that** the termination compliance value (Cₜₑᵣₘ) equals 75% to 95%, preferably 80% to 92.5%, especially preferably 85% to 91% of the reference compliance values (C_{ref}).

7. Ventilation device (10) according to one of the preceding Claims, **Characterized in that** the control device (18) is configured to calculate a volume ratio value from the quantitatively greatest difference (ΔV_{hyst-max}) occurring during the P/V maneuver for a respiratory gas pressure between the expiratory (56) and the inspiratory maneuver respiratory gas volume (54) and a difference (ΔVᵢₙₛₚ₋ₘₐₓ) between a maneuver respiratory gas volume value in an upper end-region (66) and a maneuver respiratory gas volume value in a lower end-region (68) of the respiratory gas pressure range traversed during the P/V maneuver, where the lower end-region (68) contains a start respiratory gas pressure (P_{Start}) and extends up to 1.05 times the start respiratory gas pressure (P_{Start}), and where the upper end-region (66) contains a termination respiratory gas pressure (Pₜₑᵣₘ) with which the termination compliance value (Cₜₑᵣₘ) is associated and begins at 95% of the termination respiratory gas pressure (Pₜₑᵣₘ).

8. Ventilation device (10) according to Claim 7,
**Characterized in that** the control device (18) is configured to generate, when the volume ratio value exceeds a predetermined first threshold value, an output which indicates that a recruitment procedure for recruiting the patient's lung has overwhelming prospects of success.

9. Ventilation device (10) according to one of the preceding Claims,
**Characterized in that** the control device (18) is configured to calculate a hysteresis ratio value from the size of the hysteresis area (62) which the graphs of the expiratory (56) and of the inspiratory maneuver respiratory gas volumes (54) as functions of the respiratory gas pressure between a start respiratory gas pressure (P_{Start}) at which the P/V maneuver begins and a termination respiratory gas pressure (Pₜₑᵣₘ) with which the termination compliance value (Cₜₑᵣₘ) is associated, and the size of a rectangle (64) enclosing the hysteresis area whose one corner is determined by a lower respiratory gas pressure value in a lower end-region (68) of the respiratory gas pressure range traversed during the P/V maneuver and by the respiratory gas volume value associated with the lower respiratory gas pressure value, where the lower end-region (68) contains the start respiratory gas pressure (P_{Start}) and extends up to 1.05 times the start respiratory gas pressure (P_{Start}), and whose diagonally opposite corner is determined by an upper respiratory gas pressure value in an upper end-region (66) and by the respiratory gas volume value associated with the upper respiratory gas pressure value, where the upper end-region (66) contains a termination respiratory gas pressure (Pₜₑᵣₘ) with which the termination compliance value (Cₜₑᵣₘ) is associated and begins at 95% of the termination respiratory gas pressure (Pₜₑᵣₘ).

10. Ventilation device (10) according to Claim 9,
**Characterized in that** the control device (18) is configured to generate, when the hysteresis ratio value exceeds a predetermined second threshold value, an output which indicates that a recruitment procedure for recruiting the patient's lung has overwhelming prospects of success.

## Revendications

1. Dispositif de ventilation (10) pour la ventilation artificielle d'un patient (12), comprenant :
- un dispositif de source de gaz respiratoire (15) qui fournit un gaz respiratoire inspiratoire pour la ventilation artificielle du patient (12),
- un dispositif de modification d'écoulement (16) qui est conçu pour générer et modifier la valeur d'un flux de gaz respiratoire inspiratoire (AF),
- un agencement de conduite de gaz respiratoire (30) ayant une extrémité longitudinale proximale (30a) plus proche du patient (12) en utilisation et une extrémité longitudinale distale (30b) plus éloignée du patient en utilisation, pour transporter le flux de gaz respiratoire inspiratoire (AF) de la source de gaz respiratoire (15, 62) vers le patient (12),
- un agencement de capteur de flux (44), qui est conçu pour détecter la valeur du flux de gaz respiratoire expiratoire (AF) ainsi qu'un flux de gaz respiratoire expiratoire (EF),
- un agencement de capteur de pression (27), qui est conçu pour détecter une pression du gaz respiratoire inspiratoire ainsi que du gaz respiratoire expiratoire dans l'agencement de conduite de gaz respiratoire (30),
- un dispositif de commande (18) avec une mémoire de données (19), le dispositif de commande (18) étant connecté par transmission de signaux à la mémoire de données (19), à l'agencement de capteur de flux (44) et à l'agencement de capteur de pression (27) et étant conçu pour commander la puissance de fonctionnement du dispositif de modification de flux (16) pour modifier le flux de gaz respiratoire inspiratoire (AF),
le dispositif de commande (18) étant conçu pour commander le dispositif de modification d'écoulement (16) pour effectuer une manœuvre P/V, dans laquelle du gaz respiratoire est fourni à un patient (12) dans une phase d'inspiration en augmentant la pression de gaz respiratoire, qui, dans une phase d'expiration, s'échappe passivement du patient après la fin de l'augmentation de pression, le volume de gaz respiratoire de manoeuvre présent dans le patient en raison de la manoeuvre P/V étant déterminé en association avec la pression de gaz respiratoire régnante aussi bien pendant la phase d'inspiration que pendant la phase d'expiration pour une pluralité de pressions de gaz respiratoire,
**caractérisé en ce que** le dispositif de commande (18) est conçu à cet effet,
- de déterminer, pendant la phase d'inspiration, sur la base de signaux de l'agencement de capteur de flux (44) et de l'agencement de capteur de pression (27), une séquence de valeurs de compliance qui représentent chacune une compliance pulmonaire des poumons du patient (12),
- déterminer, en fonction de la séquence de valeurs de compliance (Ci), une valeur de compliance de référence (C_{ref}),
- déterminer comme critère d'interruption pour la phase d'inspiration, à partir de la valeur de compliance de référence (C_{ref}), une valeur de compliance d'interruption (Cₜₑᵣₘ) différente en valeur absolue de la valeur de compliance de référence (C_{ref}), en tant que valeur seuil, et
- d'interrompre la phase d'inspiration lorsque la valeur de compliance d'interruption (Cₜₑᵣₘ) est atteinte ou franchie.

2. Dispositif de ventilation (10) selon la revendication 1,
**caractérisé en ce que** le dispositif de commande (18) est adapté pour calculer la séquence de valeurs de compliance (Ci) à partir de
i) un quotient d'une valeur de variation de volume (DV) associée à une pression de gaz respiratoire et d'une valeur de variation de pression (DP) associée à la même pression de gaz respiratoire, la valeur de variation de volume (DV) représentant une variation temporelle du volume de gaz respiratoire de manœuvre et la valeur de variation de pression (DV) représentant une variation temporelle de la pression de gaz respiratoire,
ou/et
ii) un quotient d'une valeur de débit associée à une pression de gaz respiratoire et de la valeur de variation de pression (DP) associée à la même pression de gaz respiratoire, la valeur de débit représentant le flux de gaz respiratoire inspiratoire (AF).

3. Dispositif de ventilation (10) selon la revendication 1 ou 2,
**caractérisé en ce que** le dispositif de commande (18) est conçu pour sélectionner la valeur de compliance (Cₘₐₓ) la plus élevée en termes de valeur comme valeur de compliance de référence (C_{ref}) parmi une série de valeurs de compliance (Ci) qui augmentent d'abord en valeur absolue et diminuent ensuite en valeur absolue.

4. Dispositif de ventilation (10) selon l'une quelconque des revendications précédentes,
**caractérisé en ce que** le dispositif de commande (18) est conçu pour déterminer, à partir d'une suite de paires de valeurs de la pression de gaz respiratoire inspiratoire et du volume de gaz respiratoire de manoeuvre associé à la pression de gaz respiratoire inspiratoire respective, un point d'inflexion (58) entre des sections (54a, 54b), courbées dans un sens de courbure différent, d'un graphique (54) représentant la suite de paires de valeurs et pour sélectionner la valeur de compliance associée à la pression de gaz respiratoire au point d'inflexion (58) comme valeur de compliance de référence (C_{ref}).

5. Dispositif de ventilation (10) selon l'une quelconque des revendications précédentes,
**caractérisé en ce que** le dispositif de commande (18) est adapté pour calculer la valeur de compliance d'interruption (Cₜₑᵣₘ) en multipliant la valeur de compliance de référence (C_{ref}) par un facteur prédéterminé ou en ajoutant la valeur de compliance de référence (C_{ref}) à un opérande prédéterminé.

6. Dispositif de ventilation (10) selon la revendication 5,
**caractérisé en ce que** la valeur de compliance d'interruption (Cterm) est comprise entre 75% et 95%, de préférence entre 80% et 92,5%, plus préférentiellement entre 85% et 91% de la valeur de compliance de référence (C_{ref}).

7. Dispositif de ventilation (10) selon l'une quelconque des revendications précédentes,
**caractérisé en ce que** le dispositif de commande (18) est adapté pour, à calculer une valeur de quotient volumique à partir de la plus grande différence (DV_{hyst-max}), en valeur absolue, apparaissant pendant la manœuvre P/V pour une pression de gaz respiratoire, entre le volume de gaz respiratoire de manœuvre expiratoire (56) et inspiratoire (54) et une valeur de différence (DVᵢₙₛₚ₋ₘₐₓ) entre une valeur de volume de gaz respiratoire de manœuvre dans une zone d'extrémité supérieure (66) et une valeur de volume de gaz respiratoire de manœuvre dans une zone d'extrémité inférieure (68) de la plage de pression de gaz respiratoire parcourue pendant la manœuvre P/V, dans lequel la région d'extrémité inférieure (68) contient une pression de gaz respiratoire de départ (P_{Start}) et s'étend jusqu'à 1,05 fois la pression de gaz respiratoire de départ (P_{Start}), et dans lequel la région d'extrémité supérieure (66) contient une pression de gaz respiratoire d'interruption (Pₜₑᵣₘ) à laquelle est associée la valeur de compliance d'interruption (Cₜₑᵣₘ) et commence à 95 % de la pression de gaz respiratoire d'interruption (Pₜₑᵣₘ).

8. Dispositif de ventilation (10) selon la revendication 7,
**caractérisé en ce que** le dispositif de commande (18) est adapté pour générer, lorsque la valeur de quotient volumique dépasse une première valeur de seuil prédéterminée, une dépense indiquant qu'une procédure de recrutement pour recruter le poumon du patient a principalement des chances de réussir.

9. Dispositif de ventilation (10) selon l'une quelconque des revendications précédentes,
**caractérisé en ce que** le dispositif de commande (18) est adapté pour calculer une valeur de quotient d'hystérésis à partir de l'aire de la surface d'hystérésis (62) représentant les graphiques des volumes de gaz respiratoire de manoeuvre expiratoire (56) et inspiratoire (54) en fonction de la pression de gaz respiratoire entre une pression de gaz respiratoire de départ (P_{Start}), à laquelle la manœuvre P/V commence, et une pression de gaz respiratoire d'interruption (Pₜₑᵣₘ), à laquelle est associée la valeur de compliance d'interruption (Cₜₑᵣₘ), et l'aire d'un rectangle (64) circonscrivant la surface d'hystérésis, dont un coin est déterminé par une valeur de pression de gaz respiratoire inférieure dans une zone d'extrémité inférieure (68) de la zone de pression de gaz respiratoire parcourue pendant la manœuvre P/V et par la valeur de volume de gaz respiratoire associée à la valeur de pression de gaz respiratoire inférieure, la zone d'extrémité inférieure (68) contenant la pression de gaz respiratoire de départ (P_{Start}) et allant jusqu'à 1,05 fois la pression de gaz respiratoire de départ (P_{Start}), et dont le coin diagonalement opposé est déterminé par une valeur de pression de gaz respiratoire supérieure dans une zone d'extrémité supérieure (66) et par la valeur de volume de gaz respiratoire associée à la valeur de pression de gaz respiratoire supérieure, la zone d'extrémité supérieure (66) contenant une pression de gaz respiratoire d'interruption (Pₜₑᵣₘ) à laquelle est associée la valeur de compliance d'interruption (Cₜₑᵣₘ), et commençant à 95 % de la pression de gaz respiratoire d'interruption (Pₜₑᵣₘ).

10. Dispositif de ventilation (10) selon la revendication 9,
**caractérisé en ce que** le dispositif de commande (18) est adapté pour générer, lorsque la valeur du quotient d'hystérésis dépasse une deuxième valeur de seuil prédéterminée, une dépense indiquant qu'une procédure de recrutement pour recruter les poumons du patient a principalement des chances de réussir.
